# EUROPEAN PATENT APPLICATION

(11) **EP 1 913 959 A1**
(43) Date of publication of application: **23.04.2008**
(21) Application number: 06021647.0
(22) Date of filing: 16.10.2006
(51) Int. Cl.: A61K 51/08, A61K 51/10

(54) **Radioactive metal complexes comprising a tridentate complexing sequence**

(71) Applicant: CIS bio international, 91192 Gif sur Yvette Cedex (FR)
(72) Inventor: Cyr, John, Fairfield, CA 94534 (US); Duatti, Adriano, 44040 Chiesuol del Fosso ( Ferrara ) (IT); Uccelli, Licia, 44100 Ferrara (IT); Boschi, Alessandra, 40128 Bologna (IT); Srinivasan, Ananth, 10629 Berlin (DE); Pasqualini, Roberto, 92140 Clamart (FR)
(74) Representative: Teipel, Stephan

(57) **Abstract**

The present invention relates to a metal complex of the general formula

MXₙ(Q)Lₘ

wherein
- M is a transition metal ion;
- X is nitrido or oxo;
- Q is a tridentate ligand comprising a peptide P, wherein P contains from 1 to 20 amino acids and optionally complexing moieties and includes a tridentate complexing sequence AB comprising at least three metal complexing donor atoms, wherein
- A and B are independently an amino acid or a complexing moiety and are linked by an amide bond, wherein the metal complexing donor atoms of AB consist of either two sulfur atoms and one nitrogen atom, or one sulfur atom and two nitrogen atoms in a sequence selected from SNS, SNN, and NNS, wherein the central nitrogen in the sequence SNS, SNN, and NNS is an amide bond nitrogen;
- L is a co-ligand selected from the group consisting of phosphine, thiol, arsine, carbonyl, and halide ligand;
- n is 0 or 1 and m = 1 or 3, with the proviso that when n = 0, m = 3, and that when n = 1, m = 1.

## Description

The invention relates to metal complexes, preferably radioactive metal complexes for use in diagnostic imaging or radiotherapy, particularly to ^{99m}Tc, ^{94m}Tc, ⁹⁹Tc, ¹⁸⁶Re, and ¹⁸⁸Re labeled complexes. The metal complexes comprise a tridentate ligand **Q** compirsing a peptide **P** including complexing sequence **AB** which contains a planar chelator to which the metal is complexed. The peptide **P** of the ligand **Q** may be linked to a targeting moiety **T** either directly or via a functional group **G.** The targeting moiety **T** comprises a structural motif capable of selectively recognizing and binding to specific target molecules in a mammalian body.

In recent years, the emphasis in nuclear medicine has shifted toward targeted molecular imaging and therapy. In this approach, localized targets such as antigens, receptors, enzymes, or pathological phenotypes are targeted by radioactive isotopes to identify or treat a disease. Because the abundance of these molecular targets is often low, it is imperative to have very specific radiopharmaceuticals with high purity and high stability.

A variety of radionuclides are known to be useful for radioimaging and radiotherapy, including metals such as ⁶⁷Ga, ⁶⁸Ga, ^{99m}Tc, ¹¹¹In, ⁹⁰Y, ¹⁷⁷Lu, ⁶⁴Cu, ¹⁸⁸Re, and ¹⁸⁶Re, and halides such as ¹⁸F, ¹²³I, and ¹³¹I. The quality of a radiopharmaceutical preparation particularly in regard to yield and stability is often dictated by the quality of the radiolabeling method (i.e. the method for attaching the radiotracer to the targeting molecule). Direct methods are know in the prior art for adding radioiodine (e.g. H.-F. Beer, et al., (1993), Nucl. Med. Biol., 20, 607) or radioactive Tc/Re (US 5,061,641) to a biological molecule. However direct methods often show instability of the radioactive compound *in vitro* (i.e. poor shelf life) and *in vivo* (i.e. release of free radioiodide or pertechnetate/perrhenate in the patient). They have the additional disadvantage that the radiolabeling is not controlled in regard to site of attachment of the radioisotope, and in fact, the radioisotope can often add in more than one location on the molecule. The biological efficacy of the molecule can be affected if the radioisotope adds to a portion of the targeting molecule critical for binding to the target.

The use of chelating agents (chelators) for radiolabeling targeting molecules with radiometals can overcome many of the disavantages of direct radiolabeling methods. The chelators form stable metal complexes that attach the radiometal more reliably and stably onto the targeting molecule. In addition, the metal chelator can be added to the targeting molecule in a more defined manner with several options for the point of attachment. Methods for labeling peptides, polypeptides, antibodies, and antibody fragments with radiometals using chelators have been disclosed in the prior art.

US 5,164,176 and US 5,250,666 disclose chelating compounds containing two sulfur donor atoms derived from two thiol groups, which together with two additional nitrogen donor atoms from amine or amide groups form an N₂S₂ tetradenate chelator. These chelators are useful for radiolabeling targeting proteins such as antibodies. The radiolabeled antibodies, or catabolites thereof, demonstrate improved biodistribution properties, including reduced localization within the intestine.

Peptide-based radiometal chelators offer the advantages of easy synthesis and modification of the chelating ligand. Hence, the chelator is easily synthesized and can be conjugated to a targeting molecule using solid phase synthesis techniques. In addition, the chelator is easily modified by exchanging the amino acids comprising the chelator, or by adding additional amino acids adjacent to the chelator sequence. Moreover, the chelator amino acid sequence can be easily incorporated into a peptide substrate in several places. Peptide based chelators may.also be incorporated into proteins during recombinant synthesis if the amino acids comprising the chelator are proteinogenic. Several examples of peptide-based chelators have been disclosed in the prior art.

US 5,849,261 discloses vasoactive intestinal peptide (VIP) receptor peptides and derivatives and analogues thereof covalently linked to peptide-based chelators for technetium and rhenium. Embodiments of such peptides labeled with γ-radiation emitting isotopes such as ^{99m}Tc, as well as methods and kits for making, radiolabeling and using such peptides to image sites in a mammalian body are described.

US 5,993,775 discloses scintigraphic imaging and radiotherapeutic agents that are radiocatively labeled peptides between 7 and 100 amino acid residues in size, comprised of a targeting amino acid sequence and a metal complexing amino acid sequence containing a single thiol. Further embodiments include technetium and rhenium complexes of these peptides.

US 6,126,916 discloses metal binding ligands comprising two amino acids and containing sulfur donor atoms from a thiol and thiosemicarbazide group. These ligands may be coupled to peptides for use in methods of diagnosis and therapy. The peptide derivatives are readily labeled with radiometals, such as isotopes of rhenium or technetium, while retaining their ability to tightly bind specific peptide receptors.

US 5,780,006 and US 5,976,495 disclose a peptide-based radiometal chelator (and radiometal complexes) comprised of 3 amino acids including a single thiol that offers the advantage that it can be incorporated into a peptide/protein-based radiophamaceutical using peptide synthesis techniques. Embodiments where the chelator is linked to targeting moieties are also disclosed, including peptides specific for sites of inflammation.

US 6,358,491 discloses somatostatin receptor binding peptides and derivatives that are covalently linked to peptide-based radiometal chelators containing a single thiol.

Mixed 3 + 1 oxotechnetium or oxorhenium complexes are likewise well known in the art. The "3 + 1" notation refers to the ligand set which complexes to the oxometal, specifically a tridentate ligand plus in addition a monodentate ligand. Tridentate ligands of the SNS, NNS, and SNN (where S and N denote sulfur or nitrogen donor atoms, respectively in the ligand) type have been reported. The SNS type complexes are typically based on bis-(2-mercatoethanol)amine ligands, using various monodentate co-ligands, typically thiols. A very broad overview on this technology is provided in WO 96/30 054.

Conjugates comprising a 3 + 1 oxometal complex plus a targeting moiety have been disclosed in which the targeting moiety binds to the metal through monodentate thiol ligands (for example a somatostatin peptide analog: J.K. Amartey, et. al., Nucl. Med. Biol., 2001, 28, 225-233). This approach is inadequate however, because the monodentate thiol ligands are susceptible to exchange with endogenous thiols like glutathione *in-vivo* (see Syhre, R., et. al., Eur. J. Nucl. Med., 1998, 25, 793-796). Hence the targeting moiety is rapidly separated from the radiolabel *in-vivo.*

M. Cattabriga et al. in J. Chem. Soc., Dalton Trans., 1998, p. 1453-1459, further report about the synthesis and structural characterization of Tc and Re complexes comprising amino acid derivatives. Specifically, they disclose a tridentate ligand consisting of the reaction product of an amino protected amino acid selected from Gly, Ala, Phe, Val, and Leu with S-methyl-2-methyldithiocarbazate. The reaction product comprises a hydrazide functionality ( N-N ) bond between the dithiocarbazate and the amino acid, and is coordinated with an SNN donor set to the metal. In this SNN structure, the central nitrogen donor atom is the nitrogen adjacent to the carbonyl group in the hydrazide functionality.

US-A-6,338,834 discloses complexation of a transition metal core with a protein. The donor atoms are in this case the SH groups of 3 cysteines and the nitrogen atom of a peptde bond. This system is in principle tetravalent, and might be considered a 3+1 system where two adjacent cysteines form a tridentate SNS donor set and a distant cysteine thiol functions as a monodentate ligand. However, it is to be noted that in this system all donor atoms belong to the same molecule, the peptide. US 6,338,834 does not suggest that good stability of a 3 + 1 complex can be achieved, wherein the monodentate ligand is free, i.e. does not belong to the same molecule.

Investigations have revealed that the thermodynamic stability of the metal chelates of the prior art is not satisfactory. The complexes of the prior art tend to dissociate thereby deteriorating the signal intensity and the contrast of the radioimaging method. The shelf life of radipharmaceuticals employing the radiometal complexes of the prior art is often limited by this instability. In order to improve the stability and increase the shelf life of the radiometal complexes, larger amounts of ligand are often used in the radiolabeling preparation. However, this can lower the biological effectiveness of the product if it is receptor-targeting, because the excess ligand can compete with the radiolabeled compound for binding sites. Excipients (e.g. anti-radiolytics) are often employed to improve the stability of radiopharmaceuticals, which adds complexity to the product formulation.

Thus, there is a demand for radioactively labeled complexes which are thermodynamically more stable than the complexes of the prior art.

It is the object of the invention to provide chelators and compounds radiolabeled using these chelators which have advantages over the chelators and radiolabeled compounds of the prior art. In particular, it is an object of the invention to provide radiopharmaceuticals possessing radiometal complexes with improved thermodynamic stability and high yields in synthesis.

It has been surprisingly found that these objectives can be attained and other deficiencies of the prior art can be overcome by a metal complex of the general formula

MXₙ(Q)Lₘ

wherein
- M is a metal;
- X is nitrido or oxo;
- Q is a tridentate ligand comprising a peptide P, wherein P contains from 1 to 20 amino acids and optionally complexing moieties and includes a tridentate complexing sequence AB comprising at least three metal complexing donor atoms, wherein
- A and B are independently selected from an amino acid and a complexing moiety and are linked by an amide bond, wherein the metal complexing donor atoms of AB consist of either two sulfur atoms and one nitrogen atom, or one sulfur atom and two nitrogen atoms in a sequence selected from SNS, SNN, and NNS, wherein the central nitrogen in the sequence SNS, SNN, and NNS is an amide bond nitrogen;
- L is a co-ligand selected from the group consisting of phosphine, thiol, arsine, carbonyl, and halide ligand;
- n is 0 or 1 and m = 1 or 3, with the proviso that when n = 0, m = 3, and that when n = 1, m = 1.

According to the above definition, the complex comprises the metal **M** and two types of ligands **Q** and **L. Q** is a tridentate ligand comprising a peptide **P.** The peptide **P** contains from 1 to 20 amino acids and optionally complexing moieties. In any case P includes a tridentate complexing sequence **AB** comprising at least three metal complexing donor atoms. **A** and **B** of the complexing sequence coordinate the metal **M** via these three donor atoms. **A** and **B** of the complexing sequence are independently an amino acid or a complexing moiety. Preferably at least one of **A** and **B** is an amino acid. In any case **A** and **B** are linked by an amide bond.

The metal complexing donor atoms of **AB** consist of either two sulfur atoms and one nitrogen atom, or one sulfur atom and two nitrogen atoms in a sequence selected from SNS, SNN, and NNS, wherein the central nitrogen in the sequence SNS, SNN, and NNS is an amide bond nitrogen. More preferably it is the amide bond nitrogen of the amide bond linking **A** and **B.**

In addition to the amino acid residue(s) comprised in **AB,** the peptide **P** may comprise one or more other amino acid residue(s) which are not directly involved in the complexation of the radioactive metal. The other amino acid residue(s) can be present
a. to adjust the physical characteristics (e.g. lipophilicity, stability) of the final complex or a radiopharmaceutical comprising the same,
b. to adjust the biodistribution or clearance of the radiopharmaceutical,
c. to provide a spacer between the targeting moiety and complexing sequence **AB,** or
d. to modify or enhance the targeting efficacy of the targeting moiety, without providing the main contribution to binding at the target, which comes from the targeting moiety.

For the purpose of the specification an amino acid residue is a constituent of the complexing sequence if at least one of its atoms is directly involved in the complexation of the radioactive metal, i.e. if at least one of its atoms is a donor atom of the metal M.

Peptide **P** may be linear, branched, or cyclic, as long as this structure does not interfere with the coordination of **M** through the complexing sequence **AB** in the peptide **P,** linear peptides being preferred.

The peptide **P** contains in general 1 to 20 amino acids, and preferably 2 to 20 amino acids, including **AB.** These may be on either one or both sides of the complexing sequence. The sequence **AB** may thus be within or at either end of the peptide **P.** Preferably, the complexing sequence **AB** is located either at the N- or the C-terminus of **P.**

The term "amino acid sequence" is defined herein as a polyamide obtainable by polycondensation of at least two amino acids, wherein "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" in the above definition.

An amide bond as used herein means any covalent bond having the structure 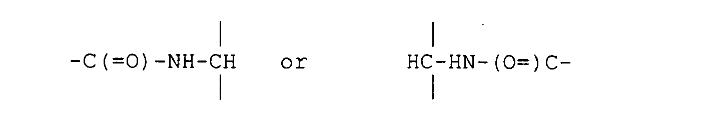 wherein the carbonyl group is provided by one molecule and the NH-group is provided by the other molecule to be joined. The amide bonds between two adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification an amino acid residue is derived from the corresponding amino acid by forming a peptide bond with another amino acid.

For the purpose of the specification an amino acid sequence may comprise naturally occurring and/or artificial amino acid residues, proteinogenic and/or non-proteinogenic amino acid residues. The non-proteinogenic amino acid residues may be further classified as (a) homo analogues of proteinogenic amino acids, (b) β-homo analogues of proteinogenic amino acid residues and (c) further non-proteinogenic amino acid residues.

Accordingly, the amino acid residues are derived from the corresponding amino acids, e.g. from
- proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
- non-proteinogenic amino acids, such as
   o homo analogues of proteinogenic amino acids wherein the sidechain has been extended by a methylene group, e.g. Homoalanine (Hal), Homoarginine (Har), Homocysteine (Hcy), Homoglutamine (Hgl), Homohistidine (Hhi), Homoisoleucine (Hil), Homoleucine (Hle), Homolysine (Hly), Homomethionine (Hme), Homophenylalanine (Hph), Homoproline (Hpr), Homoserine (Hse), Homothreonine (Hth), Homotryptophane (Htr), Homotyrosine (Hty) and Homovaline (Hva);
   o β-homo analogues of proteinogenic amino acids wherein a methylene group has been inserted between the α-carbon and the carboxyl group yielding β-amino acids, e.g. β-Homoalanine (βHal), β-Homoarginine (βHar), β-Homoasparagine (βHas), β-Homocysteine (βHcy), β-Homoglutamine (βHgl), β-Homohistidine (βHhi), β-Homoisoleucine (βHil), β-Homoleucine (βHle), β-Homolysine (βHly), β-Homomethionine (βHme), β-Homophenylalanine (βHph), β-Homoproline (βHpr), β-Homoserine (βHse), β-Homothreonine (βHth), β-Homotryptophane (βHtr), β-Homotyrosine (βHty) and β-Homovaline (βHva);
   o further non-proteinogenic amino acids, e.g. α-Aminoadipic acid (Aad), β-Aminoadipic acid (βAad), α-Aminobutyric acid (Abu), α-Aminoisobutyric acid (Aib), β-Alanine (βAla), 4-Aminobutyric acid (4-Abu), 5-Aminovaleric acid (5-Ava), 6-Aminohexanoic acid (6-Ahx), 8-Aminooctanoic acid (8-Aoc), 9-Aminononanoic acid (9-Anc), 10-Aminodecanoic acid (10-Adc), 12-Aminododecanoic acid (12-Ado), a-Aminosuberic acid (Asu), Azetidine-2-carboxylic acid (Aze), β-Cyclohexylalanine (Cha), Citrulline (Cit), Dehydroalanine (Dha), γ-Carboxyglutamic acid (Gla), α-Cyclohexylglycine (Chg), Propargylglycine (Pra), Pyroglutamic acid (Glp), α-tert-Butylglycine (Tle), 4-Benzoylphenylalanine (Bpa), δ-Hydroxylysine (Hyl), 4-Hydroxyproline (Hyp), *allo-*Isoleucine (*a*lle), Lanthionine (Lan), (1-naphthyl)alanine (1-Nal), (2-naphthyl)alanine (2-Nal), Norleucine (Nle), Norvaline (Nva), Ornithine (Orn), Phenylglycin (Phg), Pipecolic acid (Pip), Sarcosine (Sar), Selenocysteine (Sec), Statine (Sta), β-Thienylalanine (Thi), 1,2,3,4-Tetrahydroisochinoline-3-carboxylic acid (Tic), *allo*-Threonine (*a*Thr), Thiazolidine-4-carboxylic acid (Thz), γ-Aminobutyric acid (GABA), iso-Cysteine (iso-Cys), Diaminopropionic acid (Dpr), 2,4-Diaminobutyric acid (Dab), 3,4-Diaminobutyric acid (γ,βDab), Biphenylalanine (Bip), Phenylalanine substituted in para-position with - C₁-C₆-alkyl, -halide, -NH₂ or -CO₂H (Phe(4-R) wherein R = -C₁-C₆-alkyl, -halide, -NH₂, or-CO₂H); peptide nucleic acids (PNA, cf. P.E. Nielsen, Acc.Chem.Res. 32, 624-30)
- or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8 which is incorporated herein by reference.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

The peptide **P** may comprise e.g. α-amino acid residues, i.e. amino acid residues of general formula -NR^{a}-CR^{b}R^{c}-CO-, and/or β-homo amino acid residues, i.e. amino acid residues of the general formula -NR^{a}-CR^{b}R^{c}-CH₂-CO-, and/or ω-amino alkyl carboxylic acids of the general formula -NR^{a}-(CR^{b}R^{c})ₙ-CO- where n = 2-12, and/or diamino acids of the general formulas -NR^{a}-CR^{b}R^{c}-CR^{d}(NR^{e})-(CR^{f}R^{g})ₙ-CO- or -NR^{a}-(CR^{b}R^{c})ₙ-CR^{d}(NR^{e})-CO- where n = 1-10, and/or a complexing moiety, wherein R^{a}, R^{b}, R^{c}, R^{d}, R^{e}, R^{f}, and R^{g} may be any moiety capable of being covalently linked to a carbon or nitrogen atom.

When an amino acid residue comprises more than a single amino group, any of the amino groups may be involved in the peptide bond to an adjacent amino acid residue. For example, the amino acid residue of lysine may be covalently linked either through its α-amino group or its ε-amino group. The same applies to amino acid residues comprising more than a single carboxyl group, i.e. any of the carboxy groups may be involved in the peptide bond to an adjacent amino acid residue. For example, the amino acid residue of glutamic acid may be covalently linked either through its α-carboxy group or its δ-carboxy group. Branching of the amino acid sequence may be effected if, for example, both carboxy groups of glutamic acid are covalently linked to the N-terminus of 2 amino acids or amino acid sequences.

Preferably, the peptide **P** does not comprise more than 10 amino acid residues which are other than α-amino acid residues, more preferably not more than 6, most preferably not more than 4, in particular not more than 2 amino acid residues which are other than α-amino acid residues. In a preferred embodiment, all amino acid residues contained in the peptide **P** are α-amino acid residues.

Chiral amino acid residues may be present in racemic form, preferably, however, in the pure enantiomeric form. For the purpose of the specification "pure enantiomeric form" means preferably > 95% ee, more preferably > 98% ee, in particular > 99% ee.

The peptide **P** may comprise an amino acid residue including a single thiol moiety. According to the above definition, the one or two sulfur donor atom(s) of **AB** can come from single thiol-containing amino acid or from a single thiol moiety, where the single thiol moiety is defined as a non-amino acid structure or complexing moiety bearing a thiol which is capable of forming an amide bond. Preferred examples of thiol amino acids are Cys and iso-Cys. Preferred examples of the thiol complexing moiety are mecaptoacetyl (Ma), 2-mercaptopropionyl (Mp), 2-mercapto-2-methylpropionyl (Mmp), 2-mercapto-propylamino (Mpa), 2-amino-ethanethiol (Aet), 2-Amino-propanethiol (Apt), and 2-mercapto-2-methylpropylamino (Mma).

The complexing sequence **AB** of the peptide **P** is tridentate and planar. The term "planar" refers to the plane formed by donor atoms contained in the ligand backbone, and the number of donor atoms utilized by the sequence **AB** being 3 classifies the same as tridentate. Since during complexation to the metal all donor atoms of AB lie substantially in the same plane, the chelator is a planar chelator. Further atoms of the sequence **AB** do not have to but may also lie substantially in the same plane as the donor atoms.

It is well-known that transition metal complexes, and especially technetium (V) and rhenium (V) oxo or nitrido metal cores form predominantly 5-coordinate pseudo-square pyramidal complexes with planar chelators comprising 4 donor atoms. These complexes have configurations in which the 4 donor atoms of the ligand lie approximately in a plane at the base of the pyramid, and the separate fifth oxo or nitrido ligand occupies the apex of the pyramid. The planar ligand set describing the base of the pyramid can come from one tetradentate ligand, or from a combination of a tridentate ligand along with a monodentate ligand (a "3 + 1" ligand system). Other radiometals such as Cu, Pt, or Pd are also known to have planar ligand coordination environments involving 4 donor atoms. The present invention concerns a complex of the latter 3 + 1 type.

Technetium (I) and rhenium (I) metal cores form predominantly hexa-coordinate pseudo-square bipyramidal complexes with bidentate and tridentate ligands. In the case of a tridentate chelator this will result in a "3 + 3x1" ligand system. Accordingly, the complex of the invention will comprise either 1 or three ligands **L** in addition to the tridentate ligand **Q**, depending on the type of core chosen. This means, if n = 1, i.e. the core is a metal oxo or nitrido core, there is only one additional monodentate ligand **L** (m = 1), and, if n = 0, i.e. in case of low-valent hexacoordinate metal core, there are three additional monodentate ligands **L** (m = 3).

Said additional monodentate ligand L can in principle be any suitable ligand providing a donor atom. A donor atom, as used herein means any atom capable of coordinating with a electron deficient metal atom via a free electron pair. Examples of such atoms include oxygen, nitrogen, sulfur, arsenic, phosphorous, the halogenides, and compounds thereof, amongst others. Out of practical reasons the ligand L is, however, advantageously chosen from the group consisting of phosphine, thiol, arsine, carbonyl, and halide ligand. The thiol ligand can be a second ligand **Q** coordinated via its thiol residue to the transition metal M.

In one embodiment of the invention metal complex L can be a monodentate phosphine represented by the following formula: 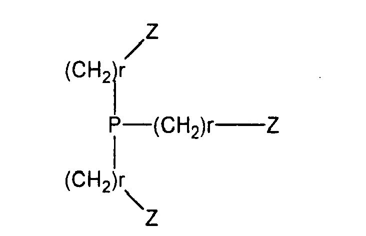 wherein each r is independently 0, 1, 2, or 3, and
Z is selected from the group consisting of H, CN, OH, O-CH₃, COOH, and C₆H₄-SO₃. More preferably, Z is not H. Most preferably, each r is the same and is 1 or 2. Similarly, most preferably, Z is CN or COOH.

In another embodiment of the invention the metal complex L can be a monodentate thiol represented by the following formula:

R₉-S⁻¹

wherein R₉ is selected from the group consisting of substituted or unsubstituted (C₁-C₆)alkyl, (C₁-C₆)hydroxyalkyl, (C₁-C₆)carboxyalkyl and phenyl, wherein the substituents are selected from the group consisting of (C₁-C₄)alkyl, (C₁-C₄)alkoxy, hydroxy, halogen and trifluoromethyl. In this case R₉ is more preferably (C₁-C₆)hydroxyalkyl or (C₁-C₆)carboxyalkyl, most preferably C₂-hydroxyalkyl or C₂-carboxyalkyl. Most preferably, L is in this embodiment mercaptoethanol or 2-mercaptoacetic acid.

Especially preferred are in one embodiment of the invention complexes, wherein n = 0, L is carbonyl or halide, and m = 3. More preferred in this case M is a radioactive transition metal and most preferably is radioactive Tc or Re.

Most preferred are complexes, wherein
either M is radioactive Tc or Re, X is oxo, n = 1 and L is a thiol co-ligand, or
M is radioactive Tc, X is nitrido, n = 1, and L is a phosphine co-ligand, or
M is radioactive Re, X is nitrido, n = 1, and L is a thiol co-ligand.

As disclosed above the complexing sequence **AB** may comprise in addition to the amino acid a complexing moiety. As used herein a "complexing moiety" is a moiety containing a thiol or amine donor atom, and having a second amine or a carboxylic acid group, allowing it to form an amide bond with the respective other of **A** and **B,** respectively.

Both of **A** and **B** can be complexing moieties. Preferably, however, only one of **A** or **B** is a complexing moiety and the other one is an amino acid. The complexing sequence **AB** is then more preferably located either at the N-terminus of the peptide P or at the C-terminus of the peptide **P.** When **AB** is located at the N-terminus, and **A** is a complexing moiety, **A** is preferably the a single thiol moiety. When **AB** is located at the C-terminus and **B** is a complexing moiety, **B** is preferably either a single thiol moiety or a diamine moiety.

In these embodiments a single thiol moiety is preferably a moiety containing a thiol group and a second functional group capable of forming an amide bond with an amino acid. More preferably the single thiol moiety is selected from HSCH₂COOH for attachment at the N-terminus, and HSCH₂CH₂NH₂ for attachment at the C-terminus of peptide **P.**

In any case, the metal complexing donor atoms of **AB** consist of either two sulfur atoms and one nitrogen atom, or one sulfur atom and two nitrogen atoms in a sequence selected from SNS, SNN, and NNS, wherein the central nitrogen in the sequence SNS, SNN, and NNS is the amide bond nitrogen of the amid bond linking **A** and **B.** The other two donor atoms are then one each provided by **A** and **B,** respectively.

The intervening atoms between the donor atoms, i.e. the intervening atoms along the perimeter of the ligand backbone between the central amide nitrogen donor atom and the two remaining donor atoms, may be any atoms capable of forming covalent bonds with the donor atoms. Preferably, along the perimeter of the ligand backbone all intervening atoms are carbon atoms. More preferably, two neighboured donor atoms are spaced apart by two or three carbon atoms.

The single thiol amino acid or a single thiol moiety containing a sulfur donor atom may have a structure represented by the following general formula: 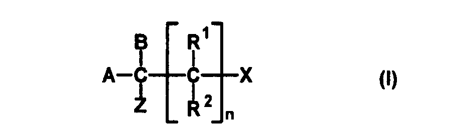 wherein
A is -CO₂H, -CONH₂, -CO₂-(pep), -CONH-(pep), or R⁴;
B is -SH, -NHR³, -N(R³)-(pep), or R⁴;
X is -SH, -NHR³, -N(R³)-(pep), or R⁴;
Z is -H or -CH₃;
n is 0, 1, or 2;
R¹, R², R³, and R⁴ are independently -H or -C₁-C₆-alkyl;
(pep) represents the remainder of the peptide **P**;
provided that
(a) B is -NHR³ or -N(R³)-(pep); X is -SH; and n is 1 or 2; or
(b) B is -SH; X is -NHR³ or -N(R³)-(pep); and n is 1 or 2; or
(c) A is -CO₂-(pep) or -CONH-(pep); B is R⁴; X is -SH and n is 0 or 1; or
(d) A is R⁴; B is -SH; and X is -N(R³)-(pep); or
(e) A is R⁴; B is -N(R³)-(pep); and X is -SH; or
(f) A is -CO₂-(pep) or -CONH-(pep); B is -SH; and X is R⁴; or
(g) A is -CO₂-(pep) or -CONH-(pep); B is -SH; X is -CH₃; Z is -CH₃; and n is 0.

Preferably, either
(a) A is -CONH-(pep); B is -N(R³)-(pep); X is -SH; Z is -H; n is 1; and R¹, R² and R³ are -H; or
(b) A is -CONH-(pep); B is -SH; X is -N(R³)-(pep); Z is -H; n is 1; and R¹, R² and R³ are -H; or
(c) A is -CONH-(pep); B is -H or -CH₃; X is -SH; Z is -H; and n is 0; or
(d) A is -CH₃; B is -SH; X is -N(R³)-(pep); Z is -H; n is 1; and R¹, R² and R³ are -H; or
(e) A is -CH₃; B is -N(R³)-(pep); X is -SH; Z is -H; n is 1; and R¹, R² and R³ are -H; or
(f) A is -CONH-(pep); B is -SH; X is -H or -CH₃; Z is -H; and n is 0; or
(g) A is -CONH-(pep); B is -SH; X is -CH₃; Z is -CH₃; and n is 0.

In another preferred embodiment the single thiol moiety has a structure represented by the above general formula wherein
(a) where B is -NHR³ or -N(R³)-(pep): X is -SH; and n is 1 or 2;
(b) where X is -NHR³ or -N(R³)-(pep): B is -SH; and n is 1 or 2;
(c) where B is R⁴: A is -CO₂H, -CONH₂, -CONH-(pep), or -CO₂-(pep); X is -SH; and n is 0 or 1;
(d) where A is R⁴ and where B is -SH: X is -NHR³ or -N(R³)-(pep); and
(e) where A is R⁴ and where X is -SH: B is -NHR³ or -N(R³)-(pep);
(f) where X is R⁴: A is -CO₂H, -CONH₂, -CONH-(pep), or -CO₂-(pep); and B is -SH; and
where Z is -CH₃: X is -CH₃; A is -CO₂H, -CONH₂, -CONH-(pep), or -CO₂-(pep); B is -SH; and n is 0.

Preferred examples of the single thiol amino acids having the structure represented by the above general formula are:

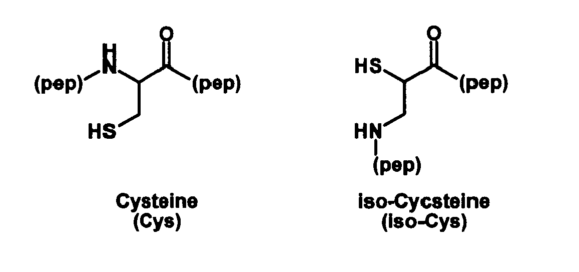

Preferred examples of the single thiol complexing moiety having a structure represented by the above general formula are:

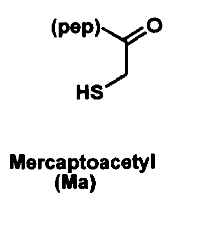

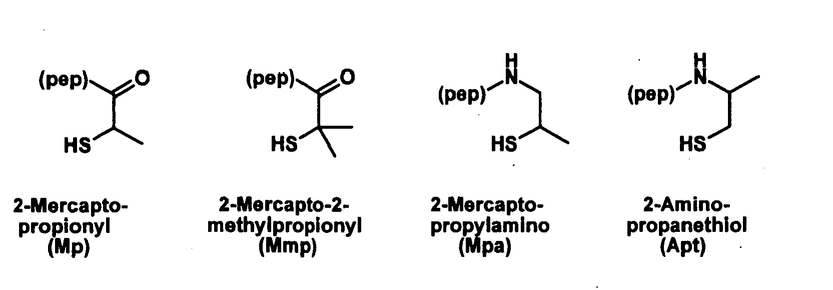

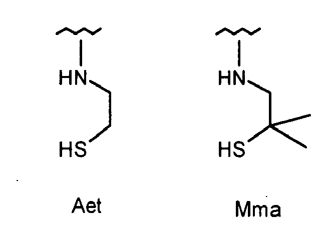

The complexing sequence **AB** may also contain an amino acid which contributes an amine nitrogen to the ligand donor set i.e. the nitrogen additional to the central nitrogen in the sequences SNN and NNS. Diamine amino acids of this type are represented by the general formulae: 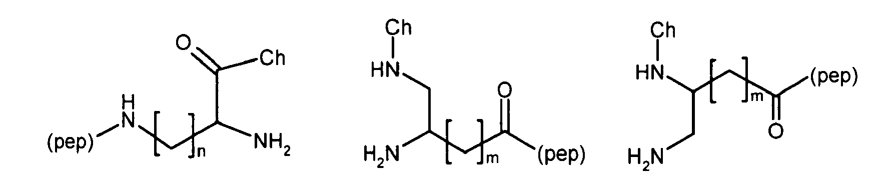 wherein (pep) represents the remainder of the peptide **P**, Ch represents the remainder of the complexing sequence **AB** and optionally in addition further remaining peptide, the index n = 1-10, and the index m = 0-10. In the most preferred embodiments of the above diamine amino acid general formulas, n = 1 and m = 0.

For the purposes of the specification, diamine amino acids are labeled with a greek letter in parentheses to signify the amine which is bonded to the remainder of the peptide or the remainder of the complexing amino acid sequence (e.g. (β)Dpr; see the structure below). If the attachment to the remainder of the peptide or to the complexing amino acid sequence in a diamine amino acid is at a normal alpha amine, then normally no greek letter is required [(α)Dpr = Dpr]. In addition, greek letters not in parentheses signify the position of the amine group (or groups) in amino acids where an amine group does not occupy the normal alpha position (e.g. 3-amino propionic acid = β-Ala). As further examples, the preferred diamine amino acids from above are:

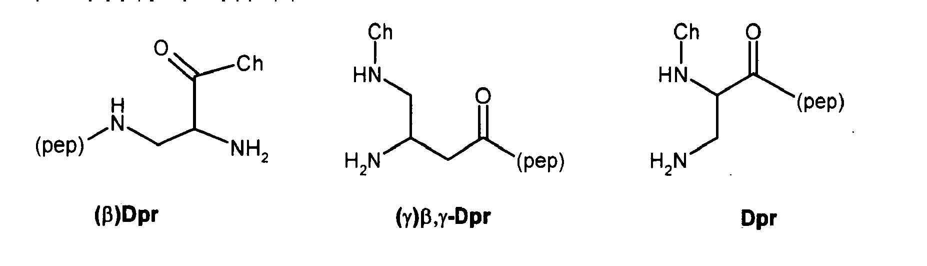

In preferred embodiments, **A** of the complexing sequence **AB** may thus be selected from the group consisting of
- a free amine N-terminal amino acid;
- a single thiol moiety;
- a thiol or thioether amino acid;
- beta-diaminopropionic acid ((β) Dpr);
- a diamino moiety of the general formula -NH(CH₂)ₓCH(NH₂)CO-, wherein x = 1-10; and
- an amino-acid the side chain of which contains a primary amine;
whereas **B** may be selected from the group consisting of:
- a thiol or thioether amino acid;
- a single thiol moiety;
- histidine;
- ethylene diamine;
- 1,3-diaminopropane;
- a diamino moiety of the general formula -NHCH(CH₂NH₂)(CH₂)_{y}CO-, wherein y = 1-10, and
- an amino-acid the side chain of which contains a primary amine.
The above selection is with the proviso that when **A** is a free amine N-terminal amino acid, Dpr, a diamino acid or an amino acid which side chain contains a primary amine, **B** is either a single thiol moiety or a thiol or thioether amino acid.

In a preferred embodiment the complexing sequence **AB** comprises and even more preferably consists of a peptidic tridentate ligand represented by one of the following formulae: 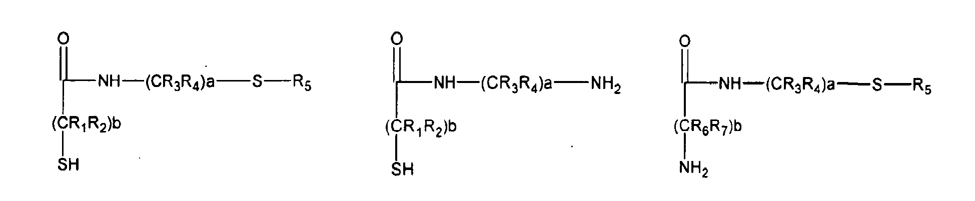 wherein
R₁, and R₂ are independently from each other and for each occurrence selected from the group consisting of -H, -NH₂, -NH-pep, -CH₂-NH₂, and -(CH₂)pNH-pep;
R₃, and R₄ are independently from each other and for each occurrence selected from the group consisting of -H, -COOH, -CO-pep, C₁-C₃ alkyl, and -(CH₂)ₚCO-pep;
R₅ is selected from the group consisting of -H, and C₁₋₃ alkyl,
R₆, and R₇ are independently selected from the group consisting of -H, -NH₂, -NH-pep, an amino acid side chain, and -(CH₂)ₚNH-pep,
pep is independently selected from the group consisting of -H, -COCH₃, -OH, -NH₂, a conjugating group (G) or a 1-18 amino acid peptide;
with the proviso that at least one of the residues R₁ to R₄ or R₆, and R₇, if present, comprises a moiety pep,
p = 1-10, a = 1-2, and b = 1-3.

Preferred examples of the complex of the invention, wherein AB is a peptide based tridentate ligand, i.e. at least one of A and B is an amino acid, are represented by one of formulae I-XIV: 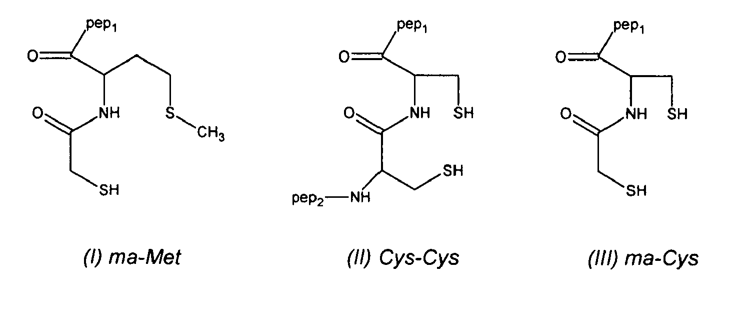 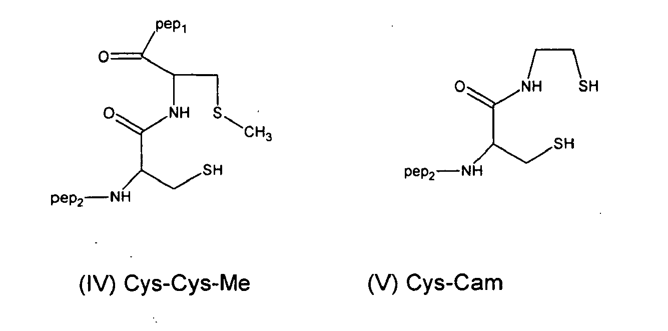 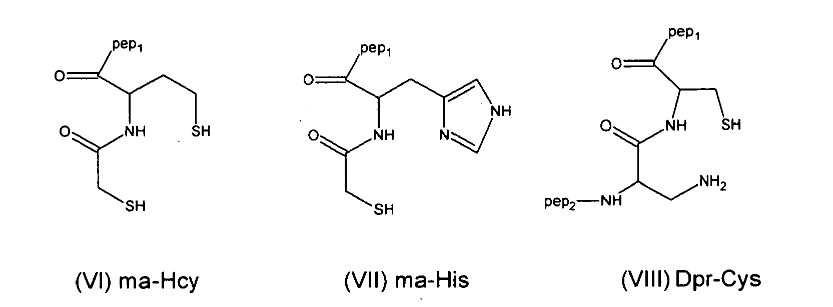 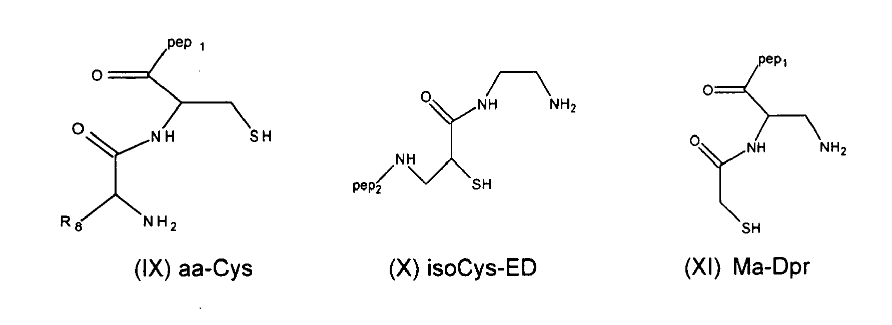 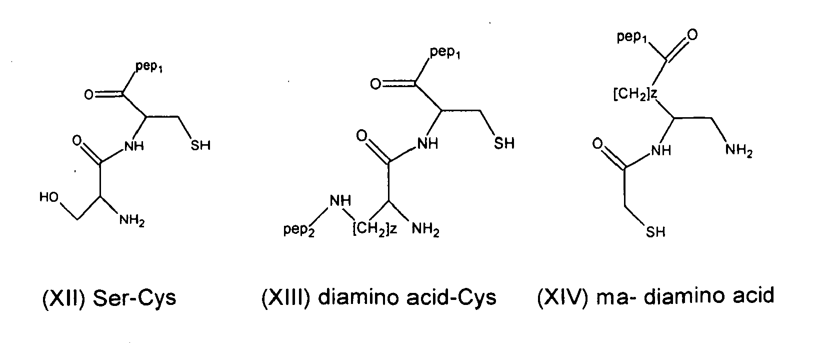 wherein
R₈ is any amino acid side chain,
z = 1-10,
pep₁ is selected from the group consisting of -OH, -NH₂, a conjugating group (G) or a 1-18 amino acid peptide;
pep₂ is selected from the group consisting of -H, -COCH₃, a conjugating group (G) or a 1-18 amino acid peptide; wherein G is defined as set out below;
with the proviso that (i) when pep, and pep₂ are both present, they when taken together contain up to and no more than 18 amino acids, and
ma = mercapto acetyl,
cam = 2-mercapto ethylamine,
Dpr = beta-diaminopropionic acid,
hCys = homocysteine, and
isoCys = isocysteine.

Preferably, in the above formulae I to XIV at least one of pep₁ or pep₂ is a 1-18 amino acid peptide.

A particularly attractive feature of peptide-based chelators such as sequences **AB** is that amino acids can be easily varied in order to modify the properties of the ligand-metal complex or moreover, a metal complex-targeting moiety conjugate. Exchanging amino acids in the chelator during solid phase synthesis effectively changes the substituents appended to the complex at the alpha positions of the amino acid carbons, while maintaining the backbone structure and donor atoms needed to complex the metal. Variation of the chelator amino acids therefore can be a useful tool during structure-activity optimization programs to improve the biological efficacy, clearance, pharmacokinetics, biodistribution, or chemical properties of metal-based peptide radiopharmaceuticals (for example: Cyr, J.E., et al., Development of an SSTR-targeting radiotherapy agent: Re-188 P2045, in Technetium, Rhenium and Other Metals in Chemistry and Nuclear Medicine, ed. M. Nicolini and U. Mazzi (Padova, Servizi Grafici Editoriali, 2002) p. 345). Modification at the chelator by amino acid substitution can also be done on tridentate chelators described here, in particular NNS chelators where **A** is any amino acid (e.g. structure IX).

In a preferred embodiment peptide **P** does not contain amino acid residues derived from β-homo amino acids, cyclic amino acids or N-methyl amino acids.

The complex of the invention comprises a metal **M,** preferably a metal capable of adopting oxidation states of 1+ to 7+. More preferred the metal **M** is a transition metal. In a preferred embodiment the metal **M** is a radioactive metal, more preferably a radioactive transition metal complexed to the complexing sequence **AB** of **Q**. The term "transition metal" is to be understood in the context of this invention to include any and all metals of the d-block metals of the periodic table, including the f-block lanthanides and actinides.

The radioactive metal may for example be selected from the group consisting of ⁴⁶Sc, ⁴⁷Sc, ⁶⁰Cu, ⁶²Cu, ⁶⁴Cu, ⁶⁷Cu, ⁶⁶Ga, ⁶⁷Ga, ⁶⁸Ga, ⁸⁶Y, ⁹⁰Y, ^{94m}Tc, ⁹⁹Tc, ^{99m}Tc, ¹⁰⁵Rh, ¹⁰³Pd, ¹¹¹In, ¹⁴²Pr, ¹⁴⁹Pm, ¹⁵³Sm, ¹⁵⁹Gd, ¹⁶⁶Ho, ¹⁷⁷Lu, ¹⁸⁶Re, ¹⁸⁸Re, ¹⁹⁹Pt, ²¹³Bi, ²²⁵Ac, ⁵²Fe, ⁶²Zn, ⁸⁹Zr, ¹⁰³Ru, ¹⁶¹Tb, and ^{117m}Sn. More preferably, the radioactive metal is selected from the group consisting of ⁶⁰Cu, ⁶²Cu, ⁶⁴Cu, ⁶⁷Cu, ^{94m}Tc, ⁹⁹Tc, ^{99m}Tc, ¹⁰³Pd, ¹⁸⁶Re, ¹⁸⁸Re and ¹⁹⁹Pt, most preferably, ^{94m}Tc, ⁹⁹Tc, ^{99m}Tc, ¹⁸⁶Re, or ¹⁸⁸Re. The latter ^{94m}Tc, ⁹⁹Tc, ^{99m}Tc, ¹⁸⁶Re, or ¹⁸⁸Re may be complexed to the planar chelator in the form of oxotechnetium, oxorhenium, nitridotechnetium or nitridorhenium.

Labeling with ^{99m}Tc is an advantage because the nuclear and radioactive properties of this isotope make it an ideal scintigraphic imaging agent. This isotope has a single photon energy of 140 keV and a radioactive half-life of about 6 hours, and is readily available from a ⁹⁹Mo-^{99m}Tc generator. Other imaging radionuclides known in the prior art have effective half-lives that are much longer (for example, ¹¹¹In, which has a half life of 67.4 h) and therefore must be handled longer for proper radioative waste decay and disposal, or pose a risk for contamination because they can react to form volatile species (for example, ¹²³I iodide). Both ¹⁸⁶Re and ¹⁸⁸Re are β-emitters and thus are suitable for therapeutic applications. ¹⁸⁶Re additionally emits a γ-ray at essentially the same energy as the y emission of ^{99m}Tc, allowing monitoring of biodistribution using the same instrumentation as is used for ^{99m}Tc imaging. ¹⁸⁸Re is available as a no carrier added isotope from a ¹⁸⁸W/¹⁸⁸Re generator analogous to the ⁹⁹Mo-^{99m}Tc generator. Those of skill will recognize that the chemical properties of technetium and rhenium are similar or substantially the same, as described in Deutsch, et al. (1986), Nucl. Med. Biol. 13, 465-477.

The peptide **P** of the ligand **Q** may form part of a conjugate **PT** which comprises the peptide **P** and a targeting moiety **T** covalently attached thereto. The peptide **P** may also be convalently attached to a functional group **G.** The functional group **G** may then link the targeting moiety T to the peptide, forming the structure **PGT.** Attachment of the targeting moiety T to the peptide **P** may occur at any position thereof, provided the attachment does not substantially interfere with the complexing of the metal **M** by the complexing sequence **AB**. More preferably, the targeting moiety and the complexing sequence **AB** are located on opposing ends of the peptide **P.**

Linking between **G** and **T** may be covalent, but may, however, also be non-covalent, e.g. via the key lock principle and mutual recognition. Examples of this type are binding pairs such as sugar/lectin, biotin/(strept)avidin, hapten/antibody and so on.

For covalent bonding, the functional group **G** is preferably selected from the group consisting of carboxyl (-CO₂H), activated carboxyl, amino (-NH₂), aldehyde (-CHO), hydrazine (-NHNH₂), semicarbacide (-NHCONHNH₂), thiosemicarbacide (-NHCSNHNH₂), isocyanate (-NCO), isothiocyanate (-NCS), imino esters (-OCNH-), maleine imide, alkenyl (-CH=CH₂), alkenylene (-CH=CH-), dienyl (-CH=CH-CH=CH₂), dienylene (-CH=CH-CH=CH-), alkynyl (-C≡CH), alkynylene (-C≡C-), α-halocarbonyl (-CO-hal), halosulfonyl (-SO₂-hal), haloacetamide (-NH-CO-CH₂-hal), acylamino (-NHCO-), mixed anhydride (-CO-O-CO-), azide (-N₃), hydroxy (-OH), carbodiimide (-N=C=N-), α,β-unsaturated carbonyl (-CH=CH-CO-) and haloacetyl (-CO-CH₂-hal), wherein halo means fluoro, chloro, bromo or iodo.

For the purpose of the specification "activated carboxyl" means a carboxyl group which is derivatised in order to facilitate the reaction with a nucleophilic group. Suitable activating groups are known to the person of ordinary skill and in this regard it can be referred to e.g. M.A. Bodanszky, "The Practice of Peptide Synthesis", Springer 1984. Examples are adducts of carboxylic acids with carbodiimides or activated esters, such as esters of hydroxybenzotriazole. Particularly preferred are activated carboxyl groups selected from the group consisting of esters of 4-nitrophenol, 3,5-dinitrophenol, pentafluorophenol, N-hydroxysuccinimide and hydroxybenzotriazole.

Preferably, there is provided the conjugate **PGT** comprising a compound **PG** as defined above and a targeting moiety **T,** wherein the targeting moiety **T** is covalently linked to the compound **PG** through a functional group **G** as defined above. The targeting moiety **T** comprises a structural motif capable of selectively recognizing and binding to specific targets in a mammalian body.

In a preferred embodiment the targeting moiety **T** is a molecule selected from the group consisting of polyacetals (e.g. polysaccharides), oligoacetals (e.g. oligosaccharides), polyesters (e.g. polynucleotides), oligoesters (e.g. oligonucleotides), polyamides (e.g. proteins), oligoamides (e.g. peptides), polyolefins (e.g. polyisoprenoids), oligoolefins (e.g. terpenes, steroids), glycoproteins, lipoproteins, antibodies, glycanes, vector amines, biogene amines, pharmaceutical drugs (e.g. antibiotics), bioactive lipids, lipoids, fatty acid esters, triglycerides, liposomes, porphyrins, texaphrins, cytochrome, inhibitors, neuramidases, prostaglandins, endothelines, alkaloids, vitamins and their analogues, hormons, anti-hormons, DNA-intercalators, nucleosides, nucleotides, lektins, peptides, antibody fragments, camelids, diabodies, affibodies, minibodies, small immunoproteins receptor agonists, receptor antagonists, statines and aptamers. One specifically preferred group of targeting moieties are the statines, especially somatostatine.

Preferably, the targeting moiety **T** comprises a targeting amino acid sequence and the conjugate **PT** or the conjugate **PGT** in total comprises at least 7 but not more than 500 amino acid residues, more preferably not more than 250, still more preferably not more than 100, most preferably not more than 50, in particular not more than 25 amino acid residues.

Preferably, the targeting moiety **T** has a molecular weight within the range of from 50 to 180,000 gmol⁻¹, more preferably from 100 to 80,000 gmol⁻¹, still more preferably from 200 to 40,000 gmol⁻¹, most preferably from 300 to 10,000 gmol⁻¹.

Preferably, the conjugate **PT** or the conjugate **PGT** has a molecular weight within the range of from 300 to 200,000 gmol⁻¹, more preferably from 500 to 100,000 gmol⁻¹, still more preferably from 700 to 50,000 gmol⁻¹, most preferably from 800 to 12,000 gmol⁻¹.

Preferably, the targeting moiety **T** is a molecule which occurs *in vivo* in an organism or which can be synthesized *in vitro.* In principle, the targeting moiety **T** is capable of interacting with a target, preferably with another molecule or molecular structure which occurs *in vivo* in an organism. Preferably, said interaction between the targeting moiety **T** and its target is based on molecular recognition resulting in a selective binding. Preferably, the binding is effected by hydrophobical interactions and/or hydrogen bonding.

Preferably, the targeting moiety **T** is capable of binding, preferably selectively binding to a cell surface receptor.

Preferably, the K_{D} binding affinity value of the targeting moiety **T** and its target is < 100 µM, more preferably < 10 µM, still more preferably < 1 µM, most preferably < 100 nM, in particular < 10 nM. The skilled person is aware of suitable methods for determining the K_{D} value of a given set of target and tageting moiety **T** under standard conditions by routine experimentation. Regarding details it can be referred to e.g. H.E. Junginger "Drug Targeting and Delivery: Concepts in Dosage Form Design", T&F STM, 1993; H. Schreier "Drug Targeting Technology: Physical, Chemical and Biological Methods", 1st ed, Marcel Dekker 2001; A.M. Hillery et al., "Drug Delivery and Targeting: For Pharmacists and Pharmaceutical Scientists", 1st ed, T&F STM, 2002 the disclosure of which being incorporated by reference herein.

In another embodiment the targeting moiety **T** does not comprise amino acid residues. Such alternative targeting moieties are e.g. sugars, or nucleic acids.

In a preferred embodiment, however, the targeting moiety **T** comprises a targeting amino acid sequence. This embodiment will be further described in the following. Preferably, the targeting moiety **T** comprises a targeting amino acid sequence selected from the group consisting of somatostatin receptor binding peptides, cyclic GPIIb/IIIa receptor binding peptides, leukocyte binding peptides, peptides derived from platelet factor 4, vasoactive intestinal peptide receptor binding peptides, neuropeptide Y receptor binding peptides, alpha-melanocyte-stimulating hormone receptor binding peptides, neurotensin receptor binding peptides, urokinase plasminogen activator receptor binding peptides, gastrin releasing peptide receptor binding peptides, α(v)β(3) receptor binding peptides, cholecystokinin receptor binding peptides, calcitonin receptor binding peptides, and chemotactic peptides.

In the case that conjugate **PT** or the conjugate **PGT** are comprised of only amino acid residues and/or single thiol moieties linked by amide bonds, the complexes can alternatively be regarded as a fusion peptide **P'** containing at least 7 but not more than 500 amino acid residues including a complexing amino acid sequence as defined above and a targeting amino acid sequence as defined above. Thus, under these circumstances it does not make a difference whether a given amino acid residue among the 7 to 500 amino acid residues of the fusion peptide **P'** belongs to the peptide **P** or the targeting moiety **T**, as long as the overall fusion peptide **P'** contains a complexing sequence **AB**, a targeting amino acid sequence and at least 7 but not more than 500 amino acid residues.

In general, when **T** is a targeting amino acid sequence, the peptide **P** comprising the complexing sequence **AB** and the targeting amino acid sequence may be covalently linked with bonds other than amide bonds. They may be covalently linked directly with one another or through the peptide backbone of a further amino acid sequence (linking amino acid sequence). However, the covalent linkage may also be effected through the sidechain of a given amino acid residue contained in the complexing sequence and the sidechain of another amino acid residue contained in the targeting amino acid sequence. Alternatively, the sidechain of an amino acid residue contained in the first of complexing sequence or targeting amino acid sequence may be covalently linked to the N-terminus or the C-terminus of the other sequence, respectively.

The invention also relates to a pharmaceutical or diagnostic composition comprising the complex of the invention as defined above and a pharmaceutically acceptable carrier.

The radioactively labeled complexes provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11th ed.

The concentration of the complexes and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable or therapeutic results are achievable.

The complexes according to the invention can be chemically synthesized *in vitro*. The peptides **P** can generally advantageously be prepared on an amino acid synthesizer. Preferably, particularly when a targeting moiety **T** is present and comprises a targeting amino acid sequence, the peptides forming fusion peptide **P'** may be synthesized sequentially, i.e. the peptide P comprising the complexing sequence **AB** and optionally the targeting amino acid sequence may be obtained by subsequently adding suitable activated and protected amino acid derivatives to the growing amino acid chain. For details regarding peptide synthesis it can be referred to e.g. B. Gutte "Peptides: Synthesis, Structures, and Applications", Academic Press, 1995; X.C. Chan et al. "Fmoc Solid Phase Peptide Synthesis: A Practical Approach", Oxford University Press, 2000; J. Jones "Amino Acid and Peptide Synthesis", 2nd ed., Oxford University Press, 2002; M. Bodanszky et al., "Principles of Peptide Synthsis", 2nd ed., Springer, 1993.

In another embodiment, the peptide **P** and the targeting moiety **T** are synthesized separately. Subsequently, the peptide **P** is covalently linked to the targeting moiety **T** during chemical in *vitro* synthesis to form the ligand **Q,** using techniques well known to those with skill in the art.

In a preferred embodiment a compound **PG** bearing a functional group **G** as defined above is ligated with a targeting moiety **T** bearing a complementary functional group which is capable of selectively reacting with the functional group **G** thereby yielding a conjugate **PGT.** For example, a maleimido group (functional group **G**) of a compound **PG** may selectively react with a thiol group (complementary functional group) of the targeting moiety, or vice versa.

In forming a complex of radioactive technetium or rhenium with the amino acid sequence to be complexed, the technetium or rhenium starting material, preferably a salt of ^{99m}Tc-, ^{94m}Tc-, or ⁹⁹Tc pertechnetate, ¹⁸⁶Re perrhenate, or ¹⁸⁸Re perrhenate, is reacted with the sequence **AB** of peptide **P** in the presence of a reducing agent; in a preferred embodiment, the reducing agent is stannous chloride. In an additional preferred embodiment, the reducing agent is a solid-phase reducing agent. Complexes and means for preparing such complexes are conveniently provided in a kit form comprising a sealed vial containing a predetermined quantity of the molecule comprising the complexing sequence AB that is to be labeled and a sufficient amount of reducing agent to label the sequence with ^{99m}Tc, ^{94m}Tc, ⁹⁹Tc, ¹⁸⁶Re, or ¹⁸⁸Re. Alternatively, the complex may be formed by reacting the complexing sequence with a pre-formed labile complex of technetium or rhenium and another compound known as a transfer ligand. This process is known as ligand exchange and is well known to those skilled in the art. The labile complex may be formed using such transfer ligands as tartrate, citrate, gluconate, glucoheptonate or mannitol, for example. Among the ^{99m}Tc pertechnate, ¹⁸⁶Re perrhenate, or ¹⁸⁸Re perrhenate salts useful with the invention are included the alkali metal salts such as the sodium salt, or ammonium salts or C₁-C₆-alkyl ammonium salts. The reaction of the amino acid sequence with technetium pertechnate or rhenium perrhenate or preformed ^{99m}Tc, ^{94m}Tc, ⁹⁹Tc, ¹⁸⁶Re, or ¹⁸⁸Re labile complex can be carried out in an aqueous medium at room temperature or with heating up to 100°C. If the metal complex has an anionic charge, it is formed in the aqueous medium in the form of a salt with a suitable cation such as sodium cation, ammonium cation, mono, di or tri C₁-C₆-alkyl amine cation, etc. Any conventional salt of the anionic complex with a pharmaceutically acceptable cation can be used in accordance with this invention.

In another embodiment of the invention as related to technetium and rhenium labeling, a free thiol on the complexing sequence can be made available (e.g. a disulfide bond is broken, or a thiol protecting group is removed) by reduction of the amino acid sequence prior to labeling. In a preferred embodiment, the reducing agent is stannous chloride. In an additional preferred embodiment, the reducing agent is a solid-phase reducing agent. The pre-reduced amino acid sequence is then labeled by reaction with ^{99m}Tc, ^{94m}Tc, ⁹⁹Tc, ¹⁸⁶Re, or ¹⁸⁸Re under reducing conditions or with pre-reduced ^{99m}Tc, ^{94m}Tc, ⁹⁹Tc, ¹⁸⁶Re, or ¹⁸⁸Re or a ^{99m}Tc, ^{94m}Tc, ⁹⁹Tc, ¹⁸⁶Re, or ¹⁸⁸Re complex.

Radioactively labeled peptides and complexes provided by the invention have a suitable amount of radioactivity. In forming ^{99m}Tc, ^{94m}Tc, ⁹⁹Tc, ¹⁸⁶Re, or ¹⁸⁸Re radioactive complexes, it is generally preferred to form radioactive complexes in solutions containing radioactivity at concentrations of from about 0.1 millicurie (mCi) to 300 mCi per ml.

Technetium-labeled complexes provided by the invention can be used for visualizing organs such as the kidney, heart or brain, for diagnosing disorders in these organs, and tumors, such as gastrointestinal tumors, myelomas and small cell lung carcinoma, endocrine tumors such as medullary thyroid carcinomas and pituitary tumors, brain tumors such as meningiomas and astrocytomas, and tumors of the prostate, breast, colon, and ovaries can also be imaged. The complexes of the invention are also used to image disease states such as coronary ischemia, thromboses, atherosclerosis, hypoxia and the like. The site imaged by the complexes of the invention will be determined by the binding specificity of the targeting moiety, e.g. the targeting amino acid sequence. Complexes of the invention labeled with therapy isotopes (e.g. ¹⁸⁶Re, ¹⁸⁸Re, ⁶⁴Cu, etc.) may be used particularly for treatment of tumors bearing the site targeted by the targeting moiety of the complexes of the invention.

In accordance with the invention, the radiolabeled complexes either as a neutral complex or as a salt with a pharmaceutically acceptable counterion are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

Preferably, the complexes of the invention exhibit a thermodynamic stability such that in aqueous solution after 8 hours more than 75% of the initial radioactive metal are still complexed, more preferably more than 80%, still more preferably more than 85%, most preferably more than 87.5%, in particular more than 90% are still complexed.

The following examples further illustrate the subject matter of the invention but should not be construed as limiting its scope.

### BRIEF DESCRIPTION OF THE DRAWINGS

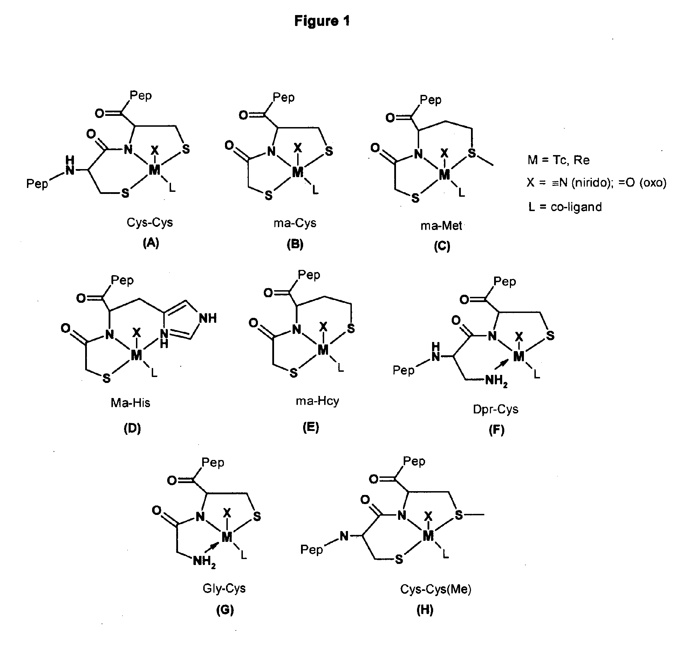

Fig. 1 is a schematic representation of various complexes of the invention, corresponding to model compounds of the following examples.

### EXAMPLES

### Example 1:

### Solid Phase Peptide Synthesis of Model Peptides

Solid phase peptide synthesis (SPPS) was carried out on a 0.1-0.25 millimole scale on an automated peptide synthesizer using 9-fluorenyl methyloxy carbonyl (Fmoc) amino-terminus protection, coupling with O-(benzotriazol-1-yl)-N,N,N',N'-bis(pentamethylene) uronium tetrafluoroborate/ hydroxybenzotriazole or dicyclohexyl carbodiimide/hydroxybenzotriazole (TBTU/ HOBT or HBTU/ HOBT), and using Rink amide resin for carboxyl-terminus amides.

N-terminal acetyl groups or mercaptoacetyl groups were introduced by using acetic acid or mercaptoacetic acid, respectively, as the last residue coupled during SPPS. Resin-bound products were routinely cleaved using a solution comprised of trifluoroacetic acid, water, thioanisole, ethanedithiol, and triethylsilane, prepared in ratios of 100:5:5:2.5:2 for 1.5-3 hours at room temperature.

Crude peptides were purified by preparative reversed phase high pressure liquid chromatography (HPLC) using gradient elution with 0.1 % trifluoroacetic acid (TFA) in water modified with acetonitrile. Acetonitrile was evaporated from the eluted fractions which were then lyophilized. The purities of the peptides were ≥ 90% by reversed phase HPLC. The identity of each product was confirmed by mass spectroscopy (ESI-MS).

Table 1 presents peptides prepared according to Example 1 along with MS results. All amino acids are in the L-form unless specified as D.

**Table 1**

| Code | Compound | MS: M + H⁺ | MW calcd |
|---|---|---|---|
| P2204 | Ac-Tyr-Gly-Cys-Cys-Gly.amide | 543.1 | 543.2 |
| P2205 | Ma-Cys-Gly-Tyr.amide | 415.0 | 415.1 |
| P2206 | Ma-(N-Me)Cys-Gly-Tyr.amide | 431.0 | 430.6 |
| P2207 | Ac-Tyr-Gly-Cys-Cam | 443.0 | 443.1 |
| P2211 | Ac-Tyr-Gly-Dpr-Cys-Gly.amide | 526.3 | 526.2 |
| P2212 | Gly-Cys-Gly-Tyr.amide | 398.2 | 398.1 |
| P2315 | Ma-Met-Gly-Tyr.amide | 443.0 | 443.1 |
| P2316 | Ma-His-Gly-Tyr.amide | 449.1 | 449.2 |
| P2317 | Ac-Tyr-Cys-Cys(Me)-Gly.amide | 500.1 | 500.1 |
| P2414 | Ma-Hcy-Gly-Tyr.amide | 428.2 | 429.0 |

### Example 2:

### Solid Phase Peptide Synthesis of Somatostatin Peptides

Example 2 is an example for the synthesis of a ligand **Q** comprising a peptide **P** covalently linked to a targeting moiety T, in this case somatostatin.

Cyclic somatostatin receptor-targeting peptides containing chelator moieties were prepared by SPPS procedures. In general the peptides were made by coupling at pH 10 a chloroacetylated, trityl-protected tetra- or pentapeptide to a thiol function on a cyclic pharmacophore hexapeptide. The trityl protecting group was subsequently removed by treatment with TFA, and the final peptides were HPLC purified as described in Example 1. A typical procedure for the coupling and final deprotection steps of the synthesis is outlined in Scheme 1.

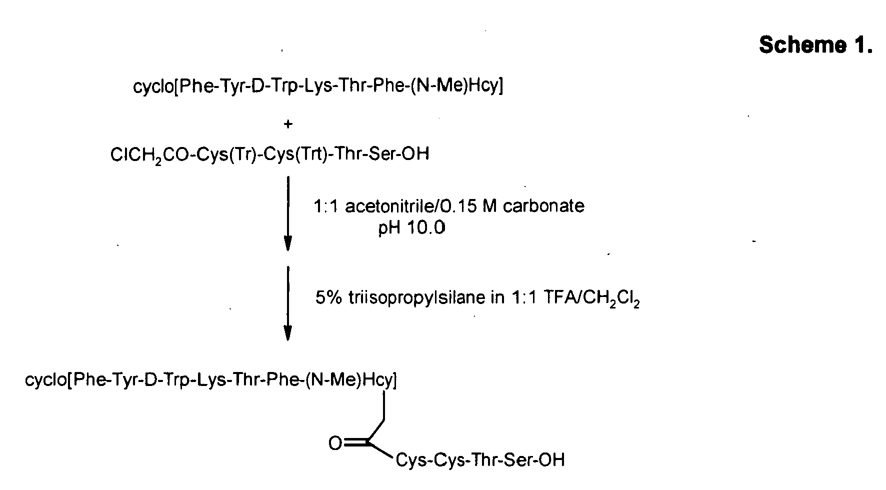

The cyclic receptor-binding pharmacophore peptides were made using SPPS with Fmoc protection and with chlorotrityl resin as the solid-phase support. Linear protected hexapeptides were synthesized by SPPS using HBTU and piperidine, and then cleaved from the resin using hexafluoroisopropanol. For the sterically demanding couplings (for example between the resin-supported N-methylhomocysteine and Fmoc-phenylalanine), [O-(7-azabenzotrazol-1-yl)-1,1,3,3-tetramethyl] uronium hexafluorophosphate (HATU) was utilized to preactivate the amino acid precursor. Homocysteine was N-methylated on the solid support by first forming the 2-nitrobenzenesulfonamide followed by deprotonation of the sulfonamide N-H with 1,3,4,6,7,8-hexahydro-1-methyl-2*H*-pyrimido[1,2-*a*]pyrimidine (MTBD) and alkylation of the nitrogen with methyl iodide. Subsequent removal of the sulfonamide with mercaptoethanol and 1,8-diazabicyclo[5.4.0]undec-7-ene (DBU) rendered the resin-supported peptide suitable for coupling to the next protected amino acid. The linear peptides were activated with HATU, cyclized, deprotected with TFA in water/triisopropylsilane/ ethanedithiol, and HPLC purified. All cyclic pharmacophore peptides were >95% pure by HPLC and had the expected MH+ peak by mass spectrometry.

The peptides **P** were also made by SPPS. For peptides in which the C-terminal functional group was either a carboxylic acid or an alcohol, chlorotrityl resin was used as the support. For peptides in which the C-terminal functional group was a carboxamide, Rink amide resin was used. Chloroacetic acid was added to the resin-supported peptides at the N-terminus. Peptides were cleaved from the resin using either hexafluoroisopropanol, or 95:5 (v/v) TFA/water. In the cases where hexafluoroisopropanol was used, the recovered peptides were fully protected. In the cases where TFA was used the *tert*-butyl-based protecting groups were removed. The trityl protecting group was selectively re-attached to the cysteine side-chain sulfhydryl group by concentrating *in vacuo* several times (redissolving the crude peptide consecutively in chloroform) to remove the TFA. All chloroacetyl peptides were >90% pure by HPLC and had the expected MH+ peak by mass spectrometry.

Table 2 presents peptides prepared according to Example 2. All amino acids are in the L-form unless specified as D.

**Table 2**

| Compound |
|---|
| cyclo-[Tyr-(D)Trp-Lys-Thr-Phe-(N-Me)Hcy]-CH₂CO.(iso)Cys-Dpr-Thr-Ser |
| cyclo-[Tyr-(D)Trp-Lys-Thr-Phe-(N-Me)Hcy]-CH₂CO.Cys-Cys-Thr-Ser |

### Example 3:

### General Method for 3 +1 Oxo Radiolabeling of the Tridentate Peptides with Tc-99m

Lyophilized kit "placebo" vials were prepared containing 5 mg sodium glucoheptonate dihydrate, 100 µg edetate disodium dihydrate, and 50 µg stannous chloride dihydrate. The formulation was adjusted to pH 7.4 prior to lyophilization.

A thiol co-ligand was dissolved in saline at 1 mg/mL and an amount of the thiol solution appropriate to acheive the desired peptide ligand: thiol ratio (*vide infra*) was added to a placebo vial. A tridentate peptide as the TFA salt was dissolved in saline at 1 mg/mL and 100 µg (100 µL) was added to the placebo vial. For pH 9 preparations, enough NaOH was added to vial to make the final preparation pH 9 (typically 25 µL 0.1 M NaOH in water).

The vial was reconstituted with technetium Tc-99m sodium pertechnetate + saline containing approximately 30 mCi Tc-99m such that the final preparation volume was 1.2 mL. The vial was incubated at room temperature for 30 minutes, or heated (at 100°C or 50°C) for 10 minutes and cooled at room temperature for 15 minutes.
The radiochemical purity (RCP) of the Tc-99m peptides was measured by gradient reversed phase HPLC using a Eurosphere 100 C-18 HPLC column and 0.1 % TFA mobile phases moderated with acetonitrile. Radioactive components were detected in the HPLC system using an in-line radiometric detector. Under the gradient conditions employed, Tc-99m glucoheptonate, Tc-99m edetate, and Tc-99m pertechnetate eluted early (between 1 and 4 minutes), while the radiolabeled peptides eluted much later (between 8 and 20 minutes). In general two main isomeric Tc-99m products were observed, and the HPLC radiochemical purity (RCP) was calculated as the sum of the % peak areas for the two products. Results for each of radiolabeled compounds are summarized in the examples that follow. All radiolabeling preparations were in addition analyzed for Tc-99m radiocolloid by TLC using ITLC strips developed in 1:1 methanol:1 M ammonium acetate in water. In all preprarations, Tc-99m radiocolloid was ≤ 5%.

### Example 4:

### Oxo Radiolabeling of the Cys-Cys Tridentate Peptide P2204 + 2-Mercaptoethanol with Tc-99m

The tridentate peptide P2204 (Ac-Tyr-Gly-Cys-Cys-Gly.amide) has an SNS donor set derived from the Cys-Cys sequence as depicted in Figure 1A. This tridentate peptide model compound along with 2-mercaptoethanol as the co-ligand L were radiolabeled with Tc-99m under various conditions and monitored by HPLC stability for up to 6 hours according to the procedures described in Example 3. Results are tabulated in Table 3.

**Table 3**

| | | | HPLC RCP (%) | | |
|---|---|---|---|---|---|
| Ratio (P:L)¹ | pH | Heat | 0.5 hours | 2 hours | 6 hours |
| 1:2 | 7.4 | 10 minutes 100°C | 18.5 | - | 23.4 |
| 1:2 | 7.4 | room temperature | 82.2 | - | 23.4 |
| 1:2 | 9 | room temperature | - | 100 | 100 |
| 1:5 | 7.4 | room temperature | 92.9 | - | 89.2 |
| 1:5 | 7.4 | 10 minutes 100°C | 93.1 | - | 87.6 |
| 1:5 | 7.4 | 10 minutes 50°C | 100 | - | 100 |

| | | | | | |
|---|---|---|---|---|---|
| ¹P = tridentate peptide; L = thiol co-ligand | | | | | |

The results indicate that when optimized the Tc-99m oxo radiolabeling of P2204 + 2-mercaptoethanol proceeds with excellent radiochemical yield.

### Example 5:

### Oxo Radiolabeling of the Ma-Cys Tridentate Peptide P2205 + 2-Mercaptoethanol or 2-Mercaptoacetic Acid with Tc-99m

The tridentate peptide P2205 (Ma-Cys-Gly-Tyr.amide) has an SNS donor set derived from the Ma-Cys sequence as depicted in Figure 1 B. This tridentate peptide model compound along with either 2-mercaptoethanol or 2-mercaptoacetic acid co-ligand were radiolabeled with Tc-99m under various conditions and monitored by HPLC stability for up to 2.5 hours according to the procedures described in Example 3. Results are tabulated in Table 4.

**Table 4**

| HPLC RCP (%) | | | | | |
|---|---|---|---|---|---|
| L | Ratio (P:L)¹ | pH | Heat | 0.5 hours | 2.5 hours |
| 2-ME² | 1:2 | 7.4 | 10 minutes 100°C | 79.0 | 76.8 |
| 2-ME | 1:2 | 9 | room temperature | 62.6 | 70.4 |
| 2-ME | 1:2 | 9 | 10 minutes 100°C | 82.7 | 81.6 |
| 2-ME | 1:5 | 7.4 | 10 minutes 100°C | 64.2 | 63.7 |
| | | | | 70.0 | 72.6 |
| 2-ME | 1:5 | 7.4 | 10 minutes 50°C | 46.9 | 55.1 |
| 2-ME | 1:5 | 7.4 | room temperature | 50.0 | 53.1 |
| 2-ME | 1:5 | 9 | room temperature | 53.1 | 58.2 |
| 2-ME | 1:5 | 9 | 10 minutes 100°C | 70.9 | 73.0 |
| 2-MA² | 1:2 | 7.4 | 10 minutes 100°C | 51.0 | 57.7 |
| 2-MA | 1:2 | 7.4 | room temperature | 51.5 | 52.1 |
| 2-MA | 1:5 | 7.4 | 10 minutes 100°C | 76.6 | 76.4 |
| 2-MA | 1:5 | 9 | 10 minutes 100°C | 81.1 | 79.9 |

| | | | | | |
|---|---|---|---|---|---|
| ¹P = tridentate peptide; L = thiol co-ligand ²2-ME = 2-mercaptoethanol; 2-MA = 2-mercaptoacetic acid | | | | | |

The results indicate that the Tc-99m oxo radiolabeling of P2205 + 2-mercaptoethanol or 2-mercaptoacetic acid proceeds with good radiochemical yield.

### Example 6:

### Oxo Radiolabeling of the Ma-Met Tridentate Peptide P2315 + 2-Mercaptoethanol with Tc-99m

The tridentate peptide P2315 (Ma-Met-Gly-Tyr.amide) has an SNS donor set derived from the Ma-Met sequence as depicted in Figure 1C. This tridentate peptide model compound along with 2-mercaptoethanol as the co-ligand L was radiolabeled with Tc-99m under various conditions and monitored by HPLC stability for up to 2.5 hours according to the procedures described in Example 3. Results are tabulated in Table 5.

**Table 5**

| HPLC RCP (%) | | | | |
|---|---|---|---|---|
| Ratio (P:L)¹ | pH | Heat | 0.5 hours | 2.5 hours |
| 1:1.2 | 9 | 10 minutes 100°C | 37.0 | 22.0 |
| 1:2 | 7.4 | 10 minutes 100°C | 62.3 | 54.9 |
| | | | 50.8 | 49.3 |
| 1:2 | 7.4 | 20 minutes 50°C | <5 | <10 |
| 1:2 | 9 | 10 minutes 100°C | 64.4 | 49.6 |
| | | | 52.5 | 41.3 |
| 1:2 | 9 | 20 minutes 50°C | 13.7 | 14.7 |
| 1:5 | 7.4 | 10 minutes 100°C | 52.3 | 45.4 |
| 1:5 | 9 | room temperature | < 5 | < 10 |

| | | | | |
|---|---|---|---|---|
| ¹P = tridentate peptide; L = thiol co-ligand | | | | |

The results indicate that the Tc-99m oxo radiolabeling of P2315 + 2-mercaptoethanol proceeds with moderate radiochemical yield.

### Example 7:

### Oxo Radiolabeling of the Ma-His Tridentate Peptide P2316 + 2-Mercaptoethanol with Tc-99m

The tridentate peptide P2316 (Ma-His-Gly-Tyr.amide) has an SNN donor set derived from the Ma-His sequence as depicted in Figure 1 D. This tridentate peptide model compound along with 2-mercaptoethanol as the co-ligand L was radiolabeled with Tc-99m under various conditions and monitored by HPLC stability for up to 2.5 hours according to the procedures described in Example 3. Results are tabulated in Table 6.

**Table 6**

| HPLC RCP (%) | | | | |
|---|---|---|---|---|
| Ratio (P:L)¹ | pH | Heat | 0.5 hours | 2.5 hours |
| 1:1.2 | 9 | 10 minutes 100°C | 79.3 | 66.0 |
| 1:2 | 7.4 | 10 minutes 100°C | - | 75.7 |
| 1:5 | 7.4 | 10 minutes 100°C | 50.4 | 42.3 |
| 1:5 | 9 | 10 minutes 100°C | 52.3 | 43.8 |

| | | | | |
|---|---|---|---|---|
| ¹P = tridentate peptide; L = thiol co-ligand | | | | |

The results indicate that the Tc-99m oxo radiolabeling of P2316 + 2-mercaptoethanol proceeds with moderate radiochemical yield.

### Example 8:

### Oxo Radiolabeling of the Ma-Hcy Tridentate Peptide P2414 + 2-Mercaptoethanol with Tc-99m

The tridentate peptide P2414 (Ma-Hcy-Gly-Tyr.amide) has an SNS donor set derived from the Ma-Hcy sequence as depicted in Figure 1 E. This tridentate peptide model compound along with 2-mercaptoethanol co-ligand was radiolabeled with Tc-99m under various conditions and monitored by HPLC stability for up to 2.5 hours according to the procedures described in Example 3. Results are tabulated in Table 7.

**Table 7**

| HPLC RCP (%) | | | | |
|---|---|---|---|---|
| Ratio (P:L)¹ | pH | Heat | 0.5 hours | 2.5 hours |
| 1:2 | 7.4 | 10 minutes 100°C | 56.2 | 57.2 |
| 1:2 | 7.4 | room temperature | 46.8 | 58.8 |
| 1:2 | 9 | room temperature | 34.4 | 34.9 |
| 1:5 | 7.4 | 10 minutes 100°C | 39.9 | 41.6 |

| | | | | |
|---|---|---|---|---|
| ¹P = tridentate peptide; L = thiol co-ligand | | | | |

The results indicate that the Tc-99m oxo radiolabeling of P2414 + 2-mercaptoethanol proceeds with low radiochemical yield.

### Example 9:

### General Methods for 3 +1 Nitrido Radiolabeling of the Tridentate Peptides with Tc-99m

### Method 1:

To 5 mg succinic dihydrazide (SDH) in a small vial was added 100 µg stannous chloride suspended in 100 µL saline. Subsequently, approximately 2.7 mCi (100 MBq) technetium Tc-99m sodium pertechnetate + saline was added in 0.9 mL such that the final preparation volume was 1.0 mL. The vial was incubated at room temperature for 15 minutes. A tridentate peptide in the form of its TFA salt was dissolved in saline at 0.02-1.0 mg/mL, and 10-500 µg (500 µL) was added to the reaction vial. Phosphine co-ligand was dissolved at 0.25-1.0 mg/mL in either a 1.3-4.0 mg/mL solution of gamma-hydroxypropylcyclodextrin in saline or in saline alone, and 0.25-1.0 mg (500 µL) was added to the vial. The vial was heated at 50-100°C for 15-60 minutes and cooled at room temperature for 10 minutes. The final pH of all preparations was pH 6-7.

The radiochemical purity (RCP) of the Tc-99m nitrido peptides was measured by gradient reversed phase HPLC using a Zorbax 300SB C-18 HPLC column and 0.1 % TFA mobile phases moderated with acetonitrile. Radioactive components were detected in the HPLC system using an in-line radiometric detector. In general two main isomeric Tc-99m products were observed, and the HPLC radiochemical purity (RCP) was calculated as the sum of the % peak areas for the two products. Results for each of radiolabeled compounds are summarized in the examples that follow. All radiolabeling preparations were in addition analyzed for Tc-99m radiocolloid + Tc-99m nitrido intermediate (R_{f} = 0.0-0.1) by TLC using C-18 plates developed in 3:7 acetonitrile:0.1% TFA in water. In all preprarations, Tc-99m radiocolloid + Tc-99m nitrido intermediate was ≤ 5%.

### Method 2:

Lyophilized SDH kit vials were prepared containing 5 mg succinic dihydrazide (SDH), 5 mg 1,2-diaminopropane-N,N,N',N' tetraacetic acid (PDTA), 100 µg stannous chloride dihydrate, 600 µg disodium dihydrogen phosphate monohydrate, and 10.9 mg disodium hydrogen phosphate heptahydrate. The formulation was adjusted to pH 7.4 prior to lyophilization.

A SDH kit vial was reconstituted with technetium Tc-99m sodium pertechnetate + saline containing approximately 20-50 mCi Tc-99m in a total volume of 1.0 mL, and the vial was incubated at room temperature for 15 minutes. A tridentate peptide in form of its TFA salt was dissolved in saline at 1 mg/mL and 25-200 µg (25-200 µL) was added to the vial. Tris-(2-carboxyethyl)phosphine (TCEP) co-ligand was dissolved at 4 mg/mL in saline, and 50-500 µg (12.5-125 µL) was added to the vial. The vial was heated at 80-100°C for 15-30 minutes and cooled at room temperature for 10 minutes. The final pH of all preparations was pH 6-7.

The radiochemical purity (RCP) of the Tc-99m nitrido peptides was measured by gradient reversed phase HPLC as described above for method 1. Results for each of radiolabeled compounds are summarized in the examples that follow. All Method 2 radiolabeling preparations were in addition analyzed for Tc-99m radiocolloid (R_{f} = 0.0-0.1) by TLC using ITLC strips developed in 1:1 methanol:0.1 M ammonium acetate in water. In all preprarations, Tc-99m radiocolloid was ≤ 5%.

### Example 10:

### Nitrido Radiolabeling of the Cys-Cys Tridentate Peptide P2204 + Tris-(2-cyanoethyl)phosphine (PCN) with Tc-99m

The tridentate peptide P2204 (Ac-Tyr-Gly-Cys-Cys-Gly.amide the sequence being depicted in Figure 1A) along with PCN co-ligand were radiolabeled with Tc-99m using various amounts of peptide and co-ligand using Method 1 described in Example 9. The phosphine co-ligand was dissolved in 4 mg/mL gamma-hydroxypropylcyclodextrin solution. All reactions were heated at 80°C for 60 minutes, and checked by HPLC within 15 minutes after heating according to the procedures described in Example 9. Results are tabulated in Table 8.

**Table 8**

| P¹ | L¹ | HPLC RCP (%) |
|---|---|---|
| 500 µg | 500 µg | 87.5 |
| 100 µg | 500 µg | 90.8 |
| 80 µg | 500 µg | 92.9 |
| 30 µg | 500 µg | 91.9 |
| 20 µg | 500 µg | 83.5 |
| 10 µg | 500 µg | 88.5 |
| 100 µg | 300 µg | 91.7 |
| 50 µg | 300 µg | 87.8 |

| | | |
|---|---|---|
| ¹P = tridentate peptide; L = phosphine co-ligand | | |

The results indicate that the Tc-99m nitrido radiolabeling of an SNS chelator comprising two thiol-containing amino acids (P2204) + a phosphine co-ligand (PCN) proceeds with excellent radiochemical yield.

### Example 11:

### Nitrido Radiolabeling of the Cys-Cys Tridentate Peptide P2204 + Tris-(3-hydroxypropyl)phosphine (P30H) with Tc-99m

The tridentate peptide P2204 (Ac-Tyr-Gly-Cys-Cys-Gly.amide; sequence depicted in Figure 1A) along with P3OH co-ligand were radiolabeled with Tc-99m using various amounts of peptide and co-ligand using Method 1 described in Example 9. The phosphine co-ligand was dissolved in 2 mg/mL gamma-hydroxypropylcyclodextrin solution. All reactions were heated at 80°C for 60 minutes, and checked by HPLC within 15 minutes after heating according to the procedures described in Example 9. Results are tabulated in Table 9.

**Table 9**

| P¹ | L¹ | HPLC RCP (%) |
|---|---|---|
| 100 µg | 1000 µg | 83.5 |
| 100 µg | 500 µg | 80.5, 88.6, 86.0² |
| 100 µg | 250 µg | 82.1 |

| | | |
|---|---|---|
| ¹P = tridentate peptide; L = phosphine co-ligand ²More than one value indicates more than one spearate preparations | | |

The results indicate that the Tc-99m nitrido radiolabeling of an SNS chelator comprising two thiol-containg amino acids (P2204) + a phosphine co-ligand (P3OH) proceeds with good radiochemical yield.

### Example 12: Nitrido Radiolabeling of the Cys-Cys Tridentate Peptide P2204 + Tris-(2-carboxyethyl)phosphine (P2COOH) with Tc-99m

The tridentate peptide P2204 (Ac-Tyr-Gly-Cys-Cys-Gly.amide; sequence depicted in Figure 1A) along with P2COOH co-ligand were radiolabeled with Tc-99m using various amounts of peptide and co-ligand using Method 1 or Method 2 described in Example 9. The phosphine co-ligand was dissolved in saline. All Method 1 reactions were heated at 100°C for 30 minutes and the Method 2 reaction was heated at 100°C for 15 minutes. The activity in the Method 2 preparation was 20 mCi. Reactions were checked by HPLC within 15 minutes after heating and at 4 hours according to the procedures described in Example 9. Results are tabulated in Table 10.

**Table 10**

| | | | | |
|---|---|---|---|---|
| HPLC RCP (%) | | | | |
| Method | P¹ | L¹ | Initial | 4 hours |
| 1 | 100 µg | 500 µg | 73.7 | |
| 1 | 100 µg | 100 µg | 78.9 | |
| 2 | 100 µg | 200 µg | 96.6 | 97.3 |

| | | | | |
|---|---|---|---|---|
| ¹P = tridentate peptide; L = phosphine co-ligand | | | | |

The results indicate that the Tc-99m nitrido radiolabeling of an SNS ligand Q comprising two thiol-containg amino acids (P2204) + a phosphine co-ligand (P2COOH) proceeds with excellent radiochemical yield.

### Example 13:

### Nitrido Radiolabeling of the Ma-Cys Tridentate Peptide P2205 + Tris-(2-cyanoethyl)phosphine (PCN) with Tc-99m

The tridentate peptide P2205 (Ma-Cys-Gly-Tyr.amide; sequence depicted in Figure 1 B) along with PCN co-ligand were radiolabeled with Tc-99m using various amounts of peptide and co-ligand using Method 1 described in Example 9. The phosphine co-ligand was dissolved in 1.3 mg/mL gamma-hydroxypropylcyclodextrin solution. All reactions were heated at 80°C for 60 minutes, and checked by HPLC within 15 minutes of heating according to the procedures described in Example 9. Results are tabulated in Table 11.

**Table 11**

| P¹ | L¹ | HPLC RCP (%) |
|---|---|---|
| 500 µg | 500 µg | 94.7 |
| 100 µg | 500 µg | 97.2 |
| 50 µg | 500 µg | 97.8 |

| | | |
|---|---|---|
| ¹P = tridentate peptide; L = phosphine co-ligand | | |

The results indicate that the Tc-99m nitrido radiolabeling of an SNS chelator comprising an N-terminal single thiol moiety and a thiol-containg amino acid (P2205) + a phosphine co-ligand (PCN) proceeds with excellent radiochemical yield.

### Example 14:

### Nitrido Radiolabeling of the Ma-Cys Tridentate Peptide P2205 + Tris-(3-hydroxypropylyl)phosphine (P30H) with Tc-99m

The tridentate peptide P2205 (Ma-Cys-Gly-Tyr.amide; sequence depicted in Figure 1 B) along with P3OH co-ligand were radiolabeled with Tc-99m using various amounts of peptide and co-ligand using Method 1 described in Example 9. The phosphine co-ligand was dissolved in 1.3 mg/mL gamma-hydroxypropylcyclodextrin solution. Reactions were heated at 80°C for 15 or 60 minutes, and checked by HPLC within 15 minutes of heating according to the procedures described in Example 9. Results are tabulated in Table 12.

**Table 12**

| P¹ | L¹ | Heat T (time) | HPLC RCP (%) |
|---|---|---|---|
| 100 µg | 500 µg | 80°C (15 minutes) | 73.1 |
| 100 µg | 500 µg | 80°C (60 minutes) | 79.6 |
| 50 µg | 500 µg | 80°C (60 minutes) | 89.7 |

| | | | |
|---|---|---|---|
| ¹P = tridentate peptide; L = phosphine co-ligand | | | |

The results indicate that the Tc-99m nitrido radiolabeling of an SNS chelator comprising an N-terminal single thiol moiety and a thiol-containg amino acid (P2205) + a phosphine co-ligand (P3OH) proceeds with very good radiochemical yield.

### Example 15:

### Nitrido Radiolabeling of the Ma-Cys Tridentate Peptide P2205 + Tris-(2-carboxyethyl)phosphine (P2COOH) with Tc-99m

The tridentate peptide P2205 (Ma-Cys-Gly-Tyr.amide; sequence depicted in Figure 1B) along with P2COOH co-ligand were radiolabeled with Tc-99m with various amounts of peptide and co-ligand using Method 2 described in Example 9. The activity in the preparations was 20 or 50 mCi. Reactions were heated at 80°C for 30 minutes or at 100°C for 15 minutes, and checked by HPLC within 10 minutes of heating (initial) and again at 2.5 hours according to the procedures described in Example 9. Results are tabulated in Table 13.

**Table 13**

| HPLC RCP (%) | | | | | |
|---|---|---|---|---|---|
| P¹ | L¹ | Activity | Heat T (time) | Initial | 2.5 hours |
| 200 µg | 500 µg | 20 mCi | 80°C (30 minutes) | 100 | 99.5 |
| 100 µg | 200 µg | 20 mCi | 80°C (30 minutes) | 94.7² | 98.1 |
| 100 µg | 200 µg | 50 mCi | 100°C (15 minutes) | 94.7² | 98.1 |
| 50 µg | 500 µg | 20 mCi | 80°C (30 minutes) | 98.9 | 99.4 |
| 25 µg | 50 µg | 50 mCi | 100°C (15 minutes) | 94.7² | 98.1 |

| | | | | | |
|---|---|---|---|---|---|
| ¹P = tridentate peptide; L = phosphine co-ligand ²Initial timepoint delayed until 1 hour | | | | | |

The results indicate that the Tc-99m nitrido radiolabeling of an SNS chelator comprising an N-terminal single thiol moiety and a thiol-containg amino acid (P2205) + a phosphine co-ligand (P2COOH) proceeds with excellent radiochemical yield.

### Example 16:

### Nitrido Radiolabeling of the Dpr-Cys Tridentate Peptide P2211 + Tris-(2-cyanoethyl)phosphine (PCN) with Tc-99m

The tridentate peptide P2211 (Ac-Tyr-Gly-Dpr-Cys-Gly.amide) has an NNS donor set derived from the Dpr-Cys sequence as depicted in Figure 1 F. This tridentate peptide model compound along with PCN co-ligand were radiolabeled with Tc-99m using various amounts of peptide and co-ligand and with various amounts of heating using Method 1 described in Example 9. The phosphine co-ligand was dissolved in 4 mg/mL gamma-hydroxypropylcyclodextrin solution. Preparations were checked by HPLC within 15 minutes of heating according to the procedures described in Example 9. Results are tabulated in Table 14.

**Table 14**

| P¹ | L¹ | Heat T (time) | HPLC RCP (%) |
|---|---|---|---|
| 500 µg | 500 µg | 50°C (30 minutes) | 91.6, 98.7² |
| 500 µg | 500 µg | 50°C (60 minutes) | 91.8 |
| 100 µg | 500 µg | 50°C (30 minutes) | 93.4 |
| 100 µg | 500 µg | 50°C (60 minutes) | 94.1, 95.7² |
| 30 µg | 500 µg | 50°C (60 minutes) | 93.5 |
| 10 µg | 500 µg | 50°C (15 minutes) | 84.7 |

| | | | |
|---|---|---|---|
| ¹P = tridentate peptide; L = phosphine co-ligand ²Two values indicates two spearate preparations | | | |

The results indicate that the Tc-99m nitrido radiolabeling of an NNS chelator comprising an amine side chain-containing amino acid and a thiol-containing amino acid (P2211) + a phosphine co-ligand (PCN) proceeds with excellent radiochemical yield.

### Example 17:

### Nitrido Radiolabeling of the Dpr-Cys Tridentate Peptide P2211 + Tris-(3-hydroxpropyl)phosphine (P30H) with Tc-99m

The tridentate peptide P2211 (Ac-Tyr-Gly-Dpr-Cys-Gly.amide; sequence depicted in Figure 1 F) along with P3OH co-ligand were radiolabeled with Tc-99m using 100 µg of peptide and 500 µg of co-ligand using Method 1 described in Example 9. The phosphine co-ligand was dissolved in 2 mg/mL gamma-hydroxypropylcyclodextrin solution. The reactions were heated at 80°C for 15 or 60 minutes, and checked by HPLC within 15 minutes of heating according to the procedures described in Example 9. Results are tabulated in Table 15.

**Table 15**

| P¹ | L¹ | Heat T (time) | HPLC RCP (%) |
|---|---|---|---|
| 100 µg | 500 µg | 80°C (15 minutes) | 80.7 |
| 100 µg | 500 µg | 80°C (60 minutes) | 78.6 |

| | | | |
|---|---|---|---|
| ¹P = tridentate peptide; L = phosphine co-ligand | | | |

The results indicate that the Tc-99m nitrido radiolabeling of an NNS chelator comprising an amine side chain-containing amino acid and a thiol-containing amino acid (P2211) + a phosphine co-ligand (P3OH) proceeds with good radiochemical yield.

### Example 18:

### Nitrido Radiolabeling of the Gly-Cys Tridentate Peptide P2212 + Tris-(2-cyanoethyl)phosphine (PCN) with Tc-99m

The tridentate peptide P2212 (Gly-Cys-Gly-Tyr.amide) has an NNS donor set derived from the N-terminal Gly-Cys sequence as depicted in Figure 1G. This tridentate peptide model compound along with PCN co-ligand were radiolabeled with Tc-99m using 500 µg of each ligand using Method 1 described in Example 9. The phosphine co-ligand was dissolved in 4 mg/mL gamma-hydroxypropylcyclodextrin solution. The reaction was heated at 80°C for 60 minutes, and checked by HPLC within 15 minutes after heating according to the procedures described in Example 9. The observed RCP was 84.9%. The results indicate that the Tc-99m nitrido radiolabeling of an NNS comprising an N-terminal amino acid and a thiol-containing amino acid (P2212) + a phosphine co-ligand (PCN) proceeds with good radiochemical yield.

### Example 19:

### Nitrido Radiolabeling of the Ma-Met Tridentate Peptide P2315 + Tris-(2-carboxyethyl)phosphine (P2COOH) with Tc-99m

The tridentate peptide P2315 (Ma-Met-Gly-Tyr.amide; sequence depicted in Figure 1C) along with P2COQH co-ligand were radiolabeled with Tc-99m with 100 µg of peptide and 200 µg co-ligand using Method 2 described in Example 9. The activity in the preparations was 20 mCi. Reactions were heated at 100°C for 15 minutes or at 80°C for 1 hour, and checked by HPLC within 10 minutes of heating (initial) and again at several timepoints according to the procedures described in Example 9. Results are tabulated in Table 16.

**Table 16**

| HPLC RCP (%) | | | | | | | |
|---|---|---|---|---|---|---|---|
| P¹ | L¹ | Heat T (time) | Initial | 3 hours | 4 hours | 6 hours | 8 hours |
| 100 µg | 200 µg | 100°C (15 minutes) | 9.5 | | 41.8 | | |
| | | | 32.2 | 42.0 | | 49.3 | 58.8 |
| 100 µg | 200 µg | 80°C (60 minutes) | 85.9 | 86.8 | | 88.9 | 92.1 |

| | | | | | | | |
|---|---|---|---|---|---|---|---|
| ¹P = tridentate chelator peptide; L = phosphine co-ligand | | | | | | | |

The results indicate that the Tc-99m nitrido radiolabeling of an SNS chelator comprising an N-terminal single thiol moiety and a thioether-containing amino acid (P2315) + a phosphine co-ligand (P2COOH) proceeds with very good radiochemical yield.

### Example 20:

### Nitrido Radiolabeling of the Ma-His Tridentate Peptide P2316 + Tris-(2-cyanoethyl)phosphine (PCN) with Tc-99m

The tridentate peptide P2316 (Ma-His-Gly-Tyr.amide; sequence depicted in Figure 1D) along with PCN co-ligand were radiolabeled with Tc-99m using 100 µg of peptide and 500 µg of co-ligand using Method 1 described in Example 9. The phosphine co-ligand was dissolved in 4 mg/mL gamma-hydroxypropylcyclodextrin solution. The reaction was heated at 80°C for 60 minutes, and checked by HPLC within 15 minutes after heating according to the procedures described in Example 9. The observed RCP was 50.8%. The results indicate that the Tc-99m nitrido radiolabeling of an SNN chelator comprising an N-terminal single thiol moiety and an imidazole-containing amino acid (P2316) + a phosphine co-ligand (PCN) proceeds with low radiochemical yield.

### Example 21:

### Nitrido Radiolabeling of the Cys-Cys(Me) Tridentate Peptide P2317 + Tris-(2-cyanoethyl)phosphine (PCN) with Tc-99m

The tridentate peptide P2317 (Ac-Tyr-Cys-Cys(Me)-Gly.amide) has an SNS donor set derived from the Cys-Cys(Me) sequence as depicted in Figure 1H. P2317 along with PCN co-ligand were radiolabeled with Tc-99m using 250 µg of peptide and 500 µg of co-ligand according to Method 1 described in Example 9. The phosphine co-ligand was dissolved in 4 mg/mL gamma-hydroxypropylcyclodextrin solution. The reaction was heated at 100°C for 30 minutes, and checked by HPLC within 15 minutes after heating according to the procedures described in Example 9. The observed RCP was 61.6%. The results indicate that the Tc-99m nitrido radiolabeling of an SNS chelator comprising a thiol-containing amino acid and a thioether-containig amino acid (P2317) + a phosphine co-ligand (PCN) proceeds with moderate radiochemical yield.

### Example 22:

### Nitrido Radiolabeling of the Cys-Cys(Me) Tridentate Peptide P2317 + Tris-(2-carboxyethyl)phosphine (P2COOH) with Tc-99m

The tridentate peptide P2317 (Ac-Tyr-Cys-Cys(Me)-Gly.amide; sequence depicted in Figure 1 H) along with P2COOH co-ligand were radiolabeled with Tc-99m using 100 µg of peptide and 200 µg of co-ligand using Method 2 described in Example 9. The activity in the preparations was 20 mCi. Reactions were heated at 100°C for 15 minutes or at 80°C for 1 hour, and checked by HPLC within 10 minutes of heating (initial) and again at several timepoints according to the procedures described in Example 9. Results are tabulated in Table 17.

**Table 17**

| HPLC RCP (%) | | | | | | | |
|---|---|---|---|---|---|---|---|
| P¹ | L¹ | Heat T (time) | Initial | 3 hours | 4 hours | 6 hours | 8 hours |
| 100 µg | 200 µg | 100°C (15 minutes) | < 5 | | 37.9 | | |
| | | | 26.8 | 34.8 | | 45.7 | 44.8 |
| 100 µg | 200 µg | 80°C (60 minutes) | 69.3 | 74.2 | | 72.7 | 75.5 |

| | | | | | | | |
|---|---|---|---|---|---|---|---|
| ¹P = tridentate peptide; L = phosphine co-ligand | | | | | | | |

The results indicate that the Tc-99m nitrido radiolabeling of an SNS chelator comprising a thiol-containing amino acid and a thioether-containing amino acid (P2317) + a phosphine co-ligand (P2COOH) proceeds with moderate radiochemical yield.

### Example 23:

### Nitrido Radiolabeling of the Ma-Hcy Tridentate Peptide P2414 + Tris-(2-carboxyethyl)phosphine (P2COOH) with Tc-99m

The tridentate peptide P2414 (Ma-Hcy-Gly-Tyr.amide; sequence depicted in Figure 1E) along with P2COOH co-ligand were radiolabeled with Tc-99m using 100 µg of peptide and 200 µg of co-ligand using Method 2 described in Example 9. The activity in the preparation was 20 mCi, and the reactions were heated at 80°C for 1 hour, and checked by HPLC within 10 minutes of heating (initial) and again at 2.5 hours according to the procedures described in Example 9. The observed RCP was 50.6 at initial and 53.9% at 2.5 hours. The results indicate that the Tc-99m nitrido radiolabeling of an SNS chelator comprising an N-terminal single thiol moiety and a thiol-containing amino acid (P2414) + a phosphine co-ligand (P2COOH) proceeds with low radiochemical yield.

### Example 24:

### Nitrido Radiolabeling of the Cys-Cys Tridentate Peptide P2204 + Tris-(2-carboxyethyl)phosphine (P2COOH) with Re-188

¹⁸⁸ReN intermediate was prepared using 1.4 mCi rhenium Re-188 sodium perrhenate as described in Example 25. To this solution was added 500 µg (1.0 mL) of P2204 peptide dissolved in saline and 500 µg (500 µL) of P2COOH co-ligand dissolved in saline, and the preparation was heated at 100°C for 30 minutes. The final pH was 4. Products were analyzed by reversed phase HPLC as described in Example 9.

Results showed 2 main product peaks with an RCP (total of both peaks) = 84.6%, and indicated that the Re-188 nitrido radiolabeling of an SNS chelator comprising two thiol-containg amino acids (P2204) + a phosphine co-ligand (P2COOH) proceeds with good radiochemical yield.

### Example 25 :

### Nitrido Radiolabeling of the Cys-Cys Tridentate Peptide P2204 + 2-Mercaptoethanol (2-ME) with Re-188

Methyl carbazate (1 mg), sodium oxalate (28 mg), stannous chloride dihydrate (0.5 mg), and ascorbic acid (5 mg) were added to a 5 mL vial. Subsequently, 50-500 µg of P2204 peptide ligand (dissolved in 0.5 mL saline), 0.5-1.0 mg 2-mercaptoethanol (dissolved in 0.5 mL saline), and 2.7 mCi rhenium Re-188 sodium perrhenate in 0.4 mL saline were added to the vial, and the preparation was heated at 100°C for 1 hour. The final pH was 4. Products were analyzed by reversed phase HPLC within 10 minutes of heating and again at several timepoints as described in Example 9. Results showed 2 main product peaks. RCP (total of both peaks) results are summarized in Table 18.

**Table 18**

| HPLC RCP (%) | | | | | | |
|---|---|---|---|---|---|---|
| P¹ | L¹ | Initial | 2 hours | 3 hours | 4 hours | 24 hours |
| 500 µg | 1000 µg | 92.6 | | | 92.8 | 93.5 |
| 500 µg | 500 µg | 81.1 | | | 83.5 | |
| 250 µg | 500 µg | 90.6 | | | 92.3 | |
| 100 µg | 1000 µg | 95.0 | | 94.7 | | 96.0 |
| 100 µg | 500 µg | 92.3 | 94.2 | | | 96.0 |
| 50 µg | 1000 µg | 95.3 | | | 94.2 | 92.5 |
| 50 µg | 500 µg | 93.6 | | 94.3 | | 96.1 |

| | | | | | | |
|---|---|---|---|---|---|---|
| ¹P = tridentate peptide; L = thiol co-ligand | | | | | | |

An additional preparation was prepared with 100 µg tridentate peptide, 1000 µg 2-ME thiol co-ligand, and higher activity (59 mCi). The initial RCP of this preparation was 93.0%. Stability of this high-activity preparation was tested after adding 10 mg ascorbic acid at pH 4 (no pH adjustment) and at pH 6.5 (adjusted with 1.0 mL of 0.5 M carbonate buffer). Results are summarized in Table 19.

**Table 19**

| HPLC RCP (%) | | | | |
|---|---|---|---|---|
| pH | Initial | 4 hours | 24 hours | 48 hours |
| 4 | 93.0 | 92.5 | 91.8 | 91.9 |
| 6.5 | 93.0 | | | 92.2 |

The results in Tables 19 and 20 indicate that the Re-188 nitrido radiolabeling of an SNS chelator comprising two thiol-containg amino acids (P2204) + a thiol co-ligand (2-ME) proceeds with excellent radiochemical yield, and furthermore, the radiolabeled complex exhibits excellent stability over 48 hours.

### Example 26 : Nitrido Radiolabeling of the Cys-Cys Tridentate Peptide P2204 + 2-Mercaptoethanol (2-ME) with Re-188, ReN core prepared with a freeze dried kit

**Preparation of the Precursor [¹⁸⁸Re≡N]²⁺ Core.** The intermediate [¹⁸⁸Re≡N]²⁺ core was prepared by adding 0.1 mL of glacial acetic acid, and 0.9 mL of Na[¹⁸⁸ReO4, 100 MBq] to a freeze-dried kit formulation composed by 5.0 mg of SDH, 10.0 mg of γ-hydroxypropylcyclodextrin, 28.0 mg of sodium oxalate, 1.0 mg of SnCl2·2H2O, and 5.0 mg of ascorbic acid. The vial was, then, heated at 100C° for 30 min. The final pH was 4.0. The radiochemical yield, determined by TLC chromatography, was (98 ± 1 %). R_{f}(¹⁸⁸Re≡N)ᵢₙₜ = 0.

**Preparation of [¹⁸⁸Re(N)(P2204)(2-ME)]** . P2204 (0.05-0.01 mg dissolved in 0.5 mL of saline), 2-mercaptoethanol (2-ME) (0.250-1.0 mg dissolved in 0.5 mL of saline) were added to the solution containing the intermediate [¹⁸⁸Re≡N]²⁺ core. The vial was heated at 100 °C for 30 min (Table 20). The final pH was 4.0. The radiochemical yield was checked by HPLC chromatography (Table 20). The HPLC chromatogram showed the presence of two main peaks at 5.7 and 7.4 min. TLC chromatography showed no significant formation of colloidal species (¹⁸⁸ReO₂). R_{f} = 0.5-0.6.

**Table 20**

| **HPLC RCP (%)** | | |
|---|---|---|
| P2204; | 2-ME | initial |
| 0.05 mg | 1.0 mg | 97.28 |
| 0.04 mg | 1.0 mg | 91.59 |
| 0.03 mg | 1.0 mg | 89.42 |
| 0.02 mg | 1.0 mg | 94.29 |
| 0.01 mg | 1.0 mg | 93.28 |
| 0.01 mg | 0.250 mg | 82.72 |
| 0.01 mg | 0.500 mg | 87.41 |

The results in Table 20 indicate that the Re-188 nitrido radiolabeling of an SNS chelator comprising two thiol-containg amino acids (P2204) + a thiol co-ligand (2-ME) proceeds with excellent radiochemical yield.

### Example 27 : Nitrido Radiolabeling of the Cys-Cys Tridentate Peptide P2204 + 2-Mercaptoethanol (2-ME) with Re-188, ReN core, high activity labeling

**High-Activity Preparation of the Precursor [¹⁸⁸Re≡N]²⁺ Core.** The intermediate [¹⁸⁸Re≡N]²⁺ core was prepared by adding 0.1 mL of glacial acetic acid and 0.9 mL of Na[¹⁸⁸ReO₄] (100 mCi) to a freeze-dried kit formulation composed by 5.0 mg of SDH, 10.0 mg of γ-hydroxypropylcyclodextrin, 28.0 mg of sodium oxalate, 1.0 mg of SnCl₂·2H₂O, and 5.0 mg of ascorbic acid. The vial was heated at 100C° for 30 min. The final pH was 4.0. The radiochemical yield, determined by TLC chromatography, was (98 ± 1%).

**High-Activity Preparation of [¹⁸⁸Re(N)(P2204)(2-ME] (1).** 0.05 mg of P2204 (dissolved in 0.5 mL of saline), 1.0 mg of 2-ME (dissolved in 0.5 mL of saline) were added to the solution containing the intermediate [¹⁸⁸Re≡N]²⁺ core. The vial was heated at 100°C for 1 hour. After cooling at room temperature, the radiochemical purity (RCP) of the preparation was checked over time by HPLC chromatography (table 21). TLC chromatography showed no significant formation of colloidal species (¹⁸⁸ReO₂).
An identical preparation was subsequently carried out, and 10.0 mg of ascorbic acid (dissolved in 0.2 ml of saline) were finally added to the reaction vial before measuring RCP (table 22)

**Table 21**

| **HPLC RCP (%)** | | | | |
|---|---|---|---|---|
| P2204; | 2-ME | initial | 24 hours | 48 hours |
| 0.05 mg | 1.0 mg | 96.78 | 63.83 | 51.85 |

**Table 22**

| **HPLC RCP (%)** | | | |
|---|---|---|---|
| P2204; | 2-ME | 24 hours | 48 hours |
| 0.05 mg | 1.0 mg | 90.45 | 83.07 |

The results in Tables 21 and 22 indicate that the Re-188 nitrido radiolabeling at high activity of an SNS chelator comprising two thiol-containg amino acids (P2204) + a thiol co-ligand (2-ME) proceeds with excellent radiochemical yield, and furthermore, the radiolabeled complex exhibits good stability over 48 hours with ascorbic acid.

### Example 28 : Nitrido Radiolabeling of the Cys-Cys Tridentate Peptide P2204 + 2-Mercaptobutyl (4-MB) with Re-188 {SNS = P2204, 4-MB = HS-CH₂CH₂CH₂CH₃}

**Preparation of [¹⁸⁸Re(N)(P2204)(4-MB] :** P2204 (0.05-0.01 mg dissolved in 0.5 mL of saline), 4-mercaptobutyl (4-MB) (1.0-2.0 mg dissolved in 0.5 mL of saline) were added to the solution containing the intermediate [¹⁸⁸Re≡N]²⁺ core. The vial was heated at 100 °C for 30 min. The final pH was 4.0. The radiochemical yield was checked over time by HPLC chromatography (Table 23). The HPLC chromatogram showed the presence of two main peaks at 37.53 and 38.12 min. TLC chromatography showed no significant formation of colloidal species (¹⁸⁸ReO₂). R_{f}= 0.38.

**Table 23**

| **HPLC RCP (%)** | | |
|---|---|---|
| P2204; | 4-MB | initial |
| 0.1 mg | 2.0 mg | 81,54 |
| 0.05 mg | 1.0 mg | 97.70 |

The results in Table 23 indicate that the Re-188 nitrido radiolabeling of an SNS chelator comprising two thiol-containg amino acids (P2204) + a thiol co-ligand (4-MB) proceeds with good radiochemical yield.

### Example 29 : The complex [¹⁸⁸Re(N)(P2205)(2-ME)] {SNS = P2205, 2-ME = HS-CH₂CH₂-OH}

**Preparation of [¹⁸⁸Re(N)(P2205)(2-ME)]** P2205 (0.05-0.01 mg dissolved in 0.5 mL of saline containing 0.65 mg of γ-hydroxypropylcyclodextrin), 2-ME (0.250-1.0 mg dissolved in 0.5 mL of saline) were added to the solution containing the intermediate [¹⁸⁸Re≡N]²⁺ core. The vial was heated for 30 min at 100°C. The final pH was 4. RCP of the preparation was monitored over time by HPLC (Table 24).The HPLC chromatogram showed the presence of two main peaks at 7.8 and 9.4 min. TLC chromatography showed no significant formation of colloidal species (¹⁸⁸ReO₂). R_{f} = 0.4.

**Table 24**

| **HPLC RCP (%)** | | | |
|---|---|---|---|
| P2205; | 2-ME | initial | 24 hours |
| 0.10 mg | 1.0 mg | 94.51 | 96.96 |
| 0.05 mg | 1.0 mg | 94.23 | 97.29 |
| 0.025 mg | 1.0 mg | 92.87 | 96.33 |
| 0.01 mg | 1.0 mg | 91.68 | 93.80 |

The results in Table 24 indicate that the Re-188 nitrido radiolabeling of P2205 + a thiol co-ligand (4-ME) proceeds with good radiochemical yield and exhibits excellent stability over 24 hours.

### Example 30 : The complex [¹⁸⁸Re(N)(P2205)(2-ME) ], High activity labelling {SNS = P2205, 2-ME = HS-CH₂CH₂-OH}

**High-Activity Preparation of the Precursor [¹⁸⁸Re≡N]²⁺ Core.** The intermediate [¹⁸⁸Re≡N]²⁺ core was prepared by adding 0.1 mL of glacial acetic acid and 0.9 mL of Na[¹⁸⁸ReO₄] (100 mCi) to a freeze-dried kit formulation composed by 5.0 mg of SDH, 10.0 mg of γ-hydroxypropylcyclodextrin, 28.0 mg of sodium oxalate, 1.0 mg of SnCl₂·2H₂O, and 5.0 mg of ascorbic acid. The vial was heated at 100°C for 30 min. The final pH was 4.0. The radiochemical yield, determined by TLC chromatography, was (98 ± 1 %).

**High-Activity Preparation of [¹⁸⁸Re(N)(P2205)(2-ME] (4).** 0.05 mg of P2205 (dissolved in 0.5 mL of saline containing 0.65 mg of γ-hydroxycyclodextrin), 1.0 mg of 2-ME (dissolved in 0.5 mL of saline) were added to the solution containing the intermediate [¹⁸⁸Re≡N]²⁺ core. The vial was heated at 100°C for 1 hour. After cooling at room temperature, RCP of the preparation was checked over time by HPLC chromatography. TLC chromatography showed no significant formation of colloidal species (¹⁸⁸ReO₂).
An identical preparation was subsequently carried out, and 10.0 mg of ascorbic acid (dissolved in 0.2 ml of saline) were finally added to the reaction vial before measuring RCP.
Results for both preparations are reported in Table 25.

**Table 25**

| **HPLC RCP (%)** | | | | |
|---|---|---|---|---|
| P2205; | 2-ME | initial | 24 hours | 48 hours without / with asc. acid |
| 0.05 mg | 1.0 mg | 86.98 | 59.19 | 36.21/92.02 |

The results in Table 25 indicate that the Re-188 nitrido radiolabeling at high activity of P2205 + a thiol co-ligand (2-ME) proceeds with good radiochemical yield, and furthermore, the radiolabeled complex exhibits good stability over 48 hours with ascorbic acid.

### Example 31 : The complex [¹⁸⁸Re(O)(P2204)(2-ME)] {SNS = P2204, 2-ME = HS-CH₂CH₂-OH}

**Preparation of [¹⁸⁸Re(O)(P2204)(2-ME].** [¹⁸⁸ReO₄]⁻ (0.90 mL,100 MBq) were added to a vial containing 10.0 mg of γ-hydroxypropylcyclodextrin, 28.0 mg of sodium oxalate, 1.0 mg of SnCl₂·2H₂O, 5.0 mg of ascorbic acid, mixed with the ligand P2204 (0.05-0.01 dissolved in 0.5 mL of saline), 1.0 mg of 2-ME (dissolved in 0.5 mL of saline), and 0.1 mL of glacial acetic acid. The vial was heated for 30 min at 100°C. HPLC chromatography showed the presence of two main peaks at 5.7 and 7.6 min. The final pH was 4. RCP and stability of the complex were checked over time by HPLC chromatography (Table 26).

**Table 26**

| **HPLC RCP (%)** | | | | |
|---|---|---|---|---|
| P2204 | 2-ME | initial | 4 hours | 24 hours |
| 0.05 mg | 1.0 mg | 94.91 | | 85.56 |
| 0.025 mg | 1.0 mg | 89.80 | 90.36 | 93.52 |
| 0.01 mg | 1.0 mg | | 94.80 | 94.35 |

The results in Table 26 indicate that the Re-188 oxo radiolabeling of P2204 + a thiol co-ligand (2-ME) proceeds with good radiochemical yield, and furthermore, the radiolabeled complex exhibits good stability.

### Example 32 : The complex [¹⁸⁸Re(O)(P2204)(2-ME)] {SNS = P2204, 2-ME = HS-CH₂CH₂-OH}, High activity

**High-activity Preparation of [¹⁸⁸Re(O)(P2204)(2-ME)]** . The preparation of this complex was carried out also using 100 mCi of initial [¹⁸⁸ReO₄]⁻ activity following the same procedure reported above. RCP of the resulting complex was checked over time by HPLC under two different experimental conditions. In a first preparation, the final solution was monitored over time without further addition of ascorbic acid, while in another preparation, 10.0 mg of ascorbic acid (dissolved in 0.2 mL of saline) were added to the final solution. Data are reported in Table 27.

**Table 27**

| **HPLC RCP (%)** | | | | |
|---|---|---|---|---|
| P2204 | 2-ME | initial | 24 hours without / with asc. acid | 48 hours without / with asc. acid |
| 0.05 mg | 21.0 mg | 89.79 | 36.21 / 85.32 | 22.74 / 79.18 |

The results in Table 27 indicate that the Re-188 oxo radiolabeling at high activity of P2204 + a thiol co-ligand (2-ME) proceeds with good radiochemical yield, and furthermore, the radiolabeled complex exhibits moderate stability over 48 hours with ascorbic acid.

## Claims

1. A metal complex of the general formula
MXₙ(Q)Lₘ
wherein
- M is a transition metal;
- X is nitrido or oxo;
- Q is a tridentate ligand comprising a peptide P, wherein P contains from 1 to 20 amino acids and optionally complexing moieties and includes a tridentate complexing sequence AB comprising at least three metal complexing donor atoms, wherein
- A and B are independently an amino acid or a complexing moiety and are linked by an amide bond, wherein the metal complexing donor atoms of AB consist of either two sulfur atoms and one nitrogen atom, or one sulfur atom and two nitrogen atoms in a sequence selected from SNS, SNN, and NNS, wherein the central nitrogen in the sequence SNS, SNN, and NNS is an amide bond nitrogen;
- L is a co-ligand selected from the group consisting of phosphine, thiol, arsine, carbonyl, and halide ligand;
- n is 0 or 1 and m = 1 or 3, with the proviso that when n = 0, m = 3, and that when n = 1, m = 1.

2. The complex of claim 1, wherein at least one of A and B is an amino acid.

3. The complex of claim 1 or claim 2, wherein the peptide P contains 2 to 20 amino acids.

4. The complex of any one of the preceding claims, wherein n = 0, L is carbonyl or halide, and m = 3.

5. The complex of any one of the preceding claims, wherein an amino acid is any compound that has a carboxylic acid functionality and an amine nitrogen having at least one free hydrogen, preferably in alpha position thereto.

6. The complex of any one of the preceding claims, wherein a complexing moiety is a moiety containing a thiol or amine donor atom, and having a second amine or a carboxylic acid group, allowing it to form an amide bond to link with the respective other of A and B.

7. The complex of any one of the preceding claims 1 and 3 to 6, wherein one of A or B is a complexing moiety and AB is either at the N-terminus of the peptide P or at the C-terminus of the peptide P.

8. The complex of claim 7, wherein AB is located at the N-terminus and A is a single thiol moiety.

9. The complex of claim 7, wherein AB is located at the C-terminus and B is either a single thiol moiety or a diamine moiety.

10. The complex of claim 8 or 9, wherein a single thiol moiety is a moiety containing a thiol group and a second functional group capable of forming an amide bond with an amino acid.

11. The complex of claim 10, wherein the single thiol moiety is selected from HSCH₂COOH for attachment at the N-terminus, and HSCH₂CH₂NH₂ for attachment at the C-terminus of peptide P.

12. The complex of any one of the preceding claims, wherein a targeting moiety T or a functional group G is covalently attached to the peptide P.

13. The complex of claim 12, wherein a conjugating moiety containing the functional group G is covalently attached to the peptide P and a targeting moiety T is linked to the functional group G.

14. The complex of any one of the preceding claims, wherein A is selected from the group consisting of
- a free amine N-terminal amino acid;
- a single thiol moiety;
- a thiol or thioether amino acid;
- beta-diaminopropionic acid (dpr);
- an diamino moiety of the general formula -NH(CH₂)ₓCH(NH₂)CO-, wherein x = 1-10; and
- an amino-acid the side chain of which contains a primary amine; and
B is selected from the group consisting of:
- a thiol or thioether amino acid;
- a single thiol moiety;
- histidine;
- ethylene diamine;
- 1,3-diaminopropane;
- an diamino moiety of the general formula -NHCH(CH₂NH₂)(CH₂)_{y}CO-, wherein y = 1-10, and
- an amino-acid the side chain of which contains a primary amine, with the proviso that
- when A is a free amine N-terminal amino acid, Dpr, a diamino acid or an amino acid which side chain contains a primary amine, B is either a single thiol moiety or a thiol or thioether amino acid.

15. The complex of any one of the preceding claims, wherein M is a radioactive transition metal ion, preferably selected from Tc-99m, Tc-99, Tc-94m, Re-188, and Re-186.

16. The complex of any one of the preceding claims, wherein:
- M is radioactive Tc or Re, X is oxo, n = 1 and L is a thiol co-ligand, or
- M is radioactive Tc, X is nitrido, n = 1 and L is a phosphine co-ligand, or
- M is radioactive Re, X is nitrido, n = 1 and L is a thiol co-ligand.

17. The complex of any one of the preceding claims 1 to 5 or 12 to 16, wherein AB is a peptidic tridentate ligand represented by one of the following formulae: 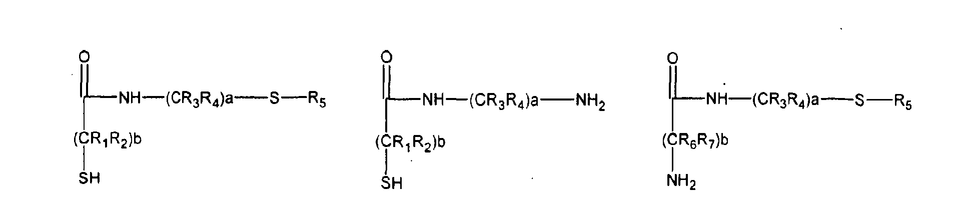 wherein
R₁, and R₂ are independently from each other and for each occurrence selected from the group consisting of -H, -NH₂, -NH-pep, -CH₂-NH₂, and -(CH₂)pNH-pep;
R₃, and R₄ are independently from each other and for each occurrence selected from the group consisting of -H, -COOH, -CO-pep, C₁-C₃ alkyl, and -(CH₂)ₚCO-pep;
R₅ is selected from the group consisting of -H, and C₁₋₃ alkyl,
R₆, and R₇ are independently selected from the group consisting of -H, -NH₂, -NH-pep, an amino acid side chain, and -(CH₂)ₚNH-pep,
pep selected from the group consisting of -H, -COCH₃, -OH, -NH₂, a conjugating group (G) or a 1-18 amino acid peptide;
with the proviso that at least one of the residues R₁ to R₄ or R₆, and R₇, if present, comprises a moiety pep,
p = 1-10, a = 1-2, and b = 1-3.

18. The complex of claim 1, wherein AB is a peptide based tridentate ligand represented by one of formulae I-XIV: 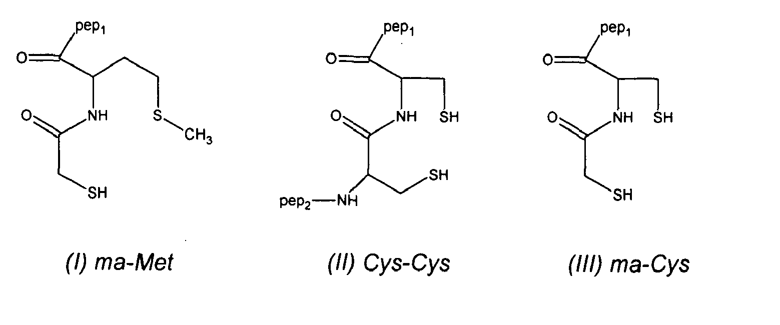 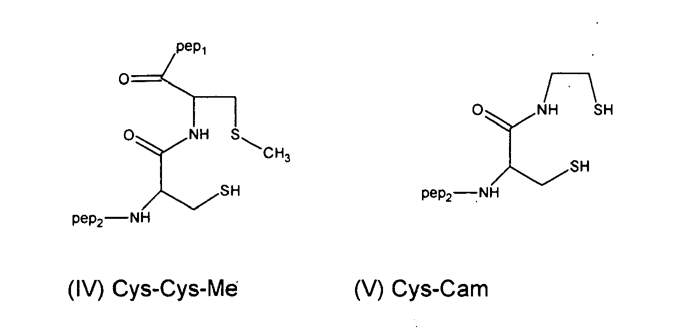 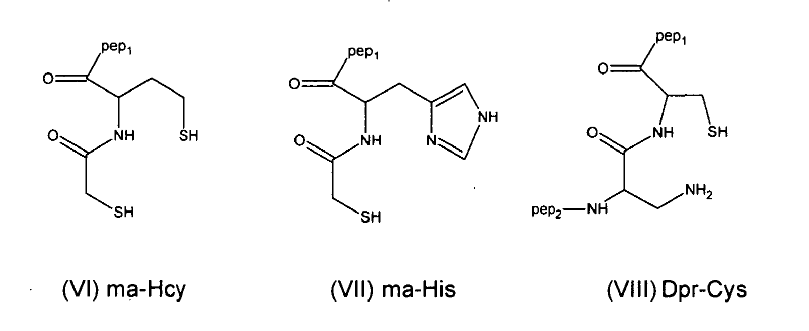 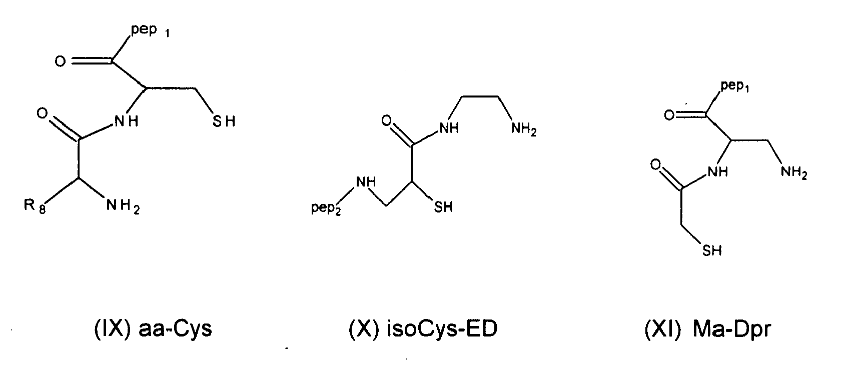 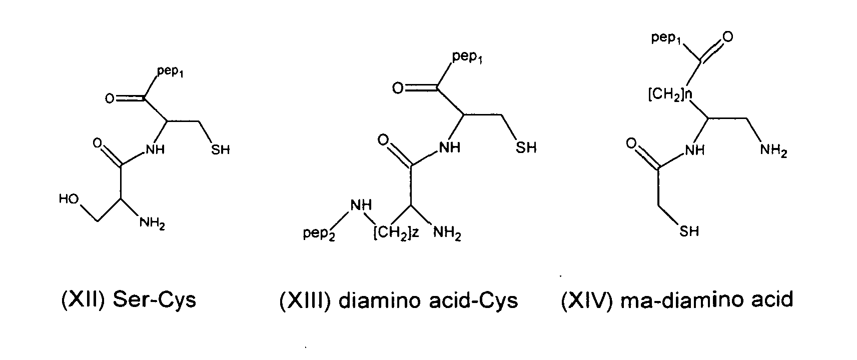 wherein
R₈ is any amino acid side chain,
z = 1-10
pep₁ is selected from the group consisting of -OH, -NH₂, a conjugating group (G) or a 1-18 amino acid peptide;
pep₂ is selected from the group consisting of -H, -COCH₃, a conjugating group (G) or a 1-18 amino acid peptide,
with the proviso that (i) when pep, and pep₂ are both present, they when taken together contain up to and no more than 18 amino acids, and
ma = mercapto acetyl,
cam = 2-mercapto ethylamine,
Dpr = beta-diaminopropionic acid,
hCys = homocysteine, and
isoCys = isocysteine.

19. The complex of claim 18, wherein at least one of pep₁ or pep₂ is a 1-18 amino acid peptide.

20. The complex of any one of the preceding claims, wherein L is a monodentate phosphine represented by the following formula: 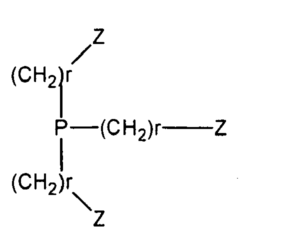 wherein
each r is independently 0, 1, 2, or 3, and
Z is selected from the group consisting of CN, OH, O-CH₃, COOH, and C₆H₄-SO₃.

21. The complex of any one of the preceding claims, wherein L is a monodentate thiol represented by the following formula:
R₉-S⁻¹
wherein
R₉ is selected from the group consisting of substituted or unsubstituted (C₁-C₆)alkyl, (C₁-C₆)hydroxyalkyl, (C₁-C₆)carboxyalkyl and phenyl, wherein the substituents are selected from the group consisting of (C₁-C₄)alkyl, (C₁-C₄)alkoxy, hydroxy, halogen and trifluoromethyl.

22. The complex of claim 18, wherein L is mercaptoethanol or 2-mercaptoacetic acid.

23. The complex of any one of the preceding claims, wherein the targeting moiety T is selected from the group consisting of polyacetals (e.g. polysaccharides), oligoacetals (e.g. oligosaccharides), polyesters (e.g. polynucleotides), oligoesters (e.g. oligonucleotides), polyamides (e.g. proteins), oligoamides (e.g. peptides), polyolefins (e.g. polyisoprenoids), oligoolefins (e.g. terpenes, steroids), glycoproteins, lipoproteins, antibodies, glycanes, vector amines, biogene amines, pharmaceutical drugs (e.g. antibiotics), bioactive lipids, lipoids, fatty acid esters, triglycerides, liposomes, porphyrins, texaphrins, cytochrome, inhibitors, neuramidases, prostaglandins, endothelines, alkaloids, vitamins and their analogues, hormones, antihormones, DNA-intercalators, nucleosides, nucleotides, lectins, peptides, antibody fragments, camelids, diabodies, minibodies, receptor agonists, receptor antagonists, and aptamers.

24. The complex of any of the preceding claims, wherein the functional group G is selected from the group consisting of carboxyl (-CO₂H), activated carboxyl, amino (-NH₂), aldehyde (-CHO), hydrazine (-NHNH₂), semicabacide (-NHCONHNH₂), thiosemicarbacide (-NHCSNHNH₂), isocyanate (-NCO), isothiocyanate (-NCS), imino esters (-OCNH-), maleine imide, alkenyl (-CH=CH₂), alkenylene (-CH=CH-), dienyl (-CH=CH-CH=CH₂), dienylene (-CH=CH-CH=CH-), alkynyl (-C≡CH), alkynylene (-C≡C-), α-halocarbonyl (-CO-hal), halosulfonyl (-SO₂-hal), haloacetamide (-NH-CO-CH₂-hal), acylamino (-NHCO-), mixed anhydride (-CO-O-CO-), azide (-N₃), hydroxy (-OH), carbodiimide (-N=C=N-), α,β-unsaturated carbonyl (-CH=CH-CO-) and haloacetyl (-CO-CH₂-hal), wherein halo means fluoro, chloro, bromo or iodo.

25. The conjugate according to claim 23 or 24, **characterized in that** the targeting moiety T comprises a targeting amino acid sequence selected from the group consisting of somatostatin receptor binding peptides, cyclic GPIIb/IIIa receptor binding peptides, leukocyte binding peptides, peptides derived from platelet factor 4, vasoactive intestinal peptide receptor binding peptides, neuropeptide Y receptor binding peptides, alpha-melanocyte-stimulating hormone receptor binding peptides, neurotensin receptor binding peptides, urokinase plasminogen activator receptor binding peptides, gastrin releasing peptide receptor binding peptides, α(v)β(3) receptor binding peptides, cholecystokinin receptor binding peptides, calcitonin receptor binding peptides, and chemotactic peptides.

26. A pharmaceutical composition comprising a complex as defined in any of the preceding claims and a pharmaceutically acceptable carrier.
